# EUROPEAN PATENT SPECIFICATION

(11) **EP 4 214 217 B1**
(45) Date of publication and mention of the grant of the patent: **18.09.2024**
(21) Application number: 21766096.8
(22) Date of filing: 14.09.2021
(51) Int. Cl.: C07H 1/00, C07H 1/06, C07H 19/048

(54) **METHODS FOR PRODUCING DIHYDRONICOTINAMIDE RIBOSIDE FROM NICOTINAMIDE RIBOSIDE CHLORIDE**
VERFAHREN ZUR HERSTELLUNG VON DIHYDRONICOTINAMIDRIBOSID AUS NICOTINAMIDRIBOSIDCHLORID
PROCÉDÉS DE PRODUCTION DE DIHYDRONICOTINAMIDE RIBOSIDE À PARTIR DE CHLORURE DE NICOTINAMIDE RIBOSIDE

(30) Priority: 15.09.2020 US 202063078526 P
(43) Date of publication of application: 26.07.2023
(73) Proprietor: Société des Produits Nestlé S.A., 1800 Vevey (CH); Cornell University, Ithaca, NY 14850 (US)
(72) Inventor: ZAREI, Amin, Ithaca, New York 14850 (US); KHAZDOOZ, Leila, Ithaca, New York 14850 (US); ENAYATINOOK, Mojtaba, Troy, AL 36081 (US); MADARSHAHIAN, Sara, Ithaca, New York 14850 (US); ABBASPOURRAD, Alireza, Ithaca, New York 14850 (US); UFHEIL, Gerhard, Chatham, New Jersey 07928 (US)
(74) Representative: Gordon, Kirsteen Helen
(86) International application number: PCT/EP2021/075245
(87) International publication number: WO 2022/058312

(56) References cited:
- WO-A1-2015/014722
- WO-A1-2017/079195
- MAKAROV M. V. ET AL: "Scalable syntheses of traceable ribosylated NAD + precursors", ORGANIC & BIOMOLECULAR CHEMISTRY, vol. 17, no. 38, 2 October 2019 (2019-10-02), pages 8716 - 8720, XP055812690, ISSN: 1477-0520, Retrieved from the Internet <URL:https://pubs.rsc.org/en/content/articlepdf/2019/ob/c9ob01981b> DOI: 10.1039/C9OB01981B

## Description

### BACKGROUND

The present disclosure generally relates to methods for producing dihydronicotinamide riboside from nicotinamide riboside chloride and related purification and stabilization methods.

Nicotinamide adenine dinucleotide (NAD⁺), and its reduced form 1,4-dihydronicotinamide adenine dinucleotide (NADH), are key molecules in energy metabolism and mitochondrial function by electron transfer. Moreover, NAD⁺ is a crucial cofactor in a number of non-redox reactions, by causing adenosine diphosphate ribose (ADP-ribose) to enzymatically catalyze the functions of the two essential protein families, sirtuins (SIRTs) and poly (ADP-ribose) polymerases (PARPs). The sirtuins have several key roles maintaining nuclear, mitochondrial, cytoplasmic or metabolic homeostasis. The most important roles of PARPs are repairing DNA and maintaining chromatin structure and function. Aging, and some disruptive factors such as an acute injury or chronic metabolic or inflammatory conditions, can cause levels of NAD⁺ to severely decline.

The drop of NAD⁺ leads to the decrease of energy production which, subsequently, impairs the cellular function, cellular homeostasis, and immune cell function. This phenomenon becomes even more important when the cells are damaged by ordinary environmental factors, because repairing damage requires a large amount of NAD⁺. If the injury is severe, the damaged cells will not have enough stored energy to provide the NAD⁺ needed for homeostasis maintenance, and so the damage becomes irreversible. The brain, heart, liver, kidneys and skeletal muscles are the organs with higher numbers of mitochondria, therefore, these vital organs are more susceptible to NAD⁺ depletion. Therefore, an energy-rich NAD⁺ precursor is needed to keep the cell with damaged tissue at normal levels of energy. Nicotinamide riboside (NR), nicotinic acid (niacin), and nicotinamide are commercially available, natural compounds used as nutritional supplements to increase the concentrations of NAD⁺.

NR is a more efficient NAD⁺ precursor in comparison to niacin and nicotinamide because it is metabolized to NAD⁺ in mammalian cells in fewer steps (**Scheme 1**). Studies show that taking NR as a supplement is effective for stimulating NAD⁺ metabolism and can boost the level of NAD⁺ by 60 percent.

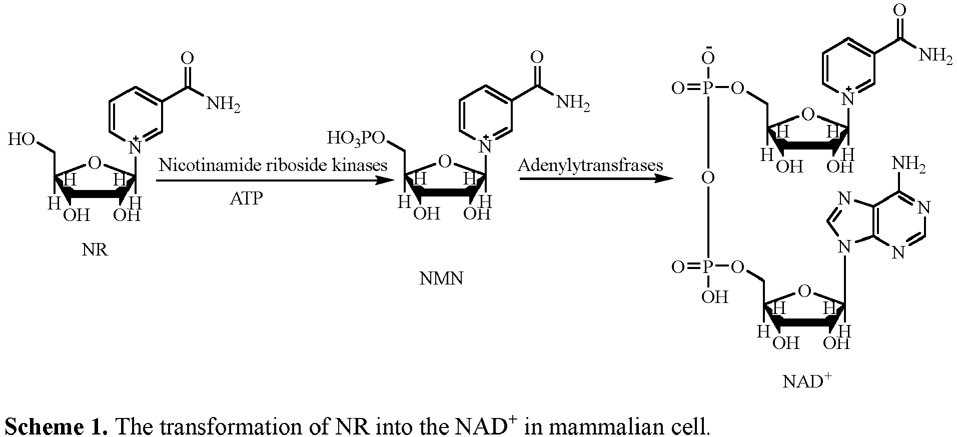

Although NR can increase the NAD⁺ level of cell and improve cell health, NR must be taken in large quantities to be effective. Recently, Sauve et al. have synthesized 1,4-dihydronicotinamide riboside (NRH) and demonstrated that this compound is a potent NAD⁺ concentration enhancer in both in vitro and in vivo conditions. They found that, after administration of NRH to mammalian cells, it increased the NAD⁺ concentration by 2.5-10-fold over control values in just one hour. Their findings demonstrate that the use of NRH is more effective than either NR or NMN. Moreover, NRH considerably enhances the NAD⁺/NADH ratio in the cultured cells without induction of apoptotic markers or a substantial increases in lactate levels in cells. More recently, Canto et al. have found that, contrary to the NR pathway, NRH uses different steps and enzymes to synthesize NAD⁺. That explains why NRH is a more effective and a faster NAD⁺ precursor compared to the NR in mammalian cells. The same researchers have also demonstrated, in experiments with mice, that NRH is orally bioavailable as an NAD⁺ precursor and prevents cisplatin-induced, acute kidney injury. In addition to increasing NAD⁺ levels, NRH can also deplete some genotoxins such as hydrogen peroxide and methylmethane sulfonate. As a result, the mouse cells treated with NRH are resistant to cell death.

There are very few methods for the preparation of NRH. This compound can be prepared from dihydronicotinamide mononucleotide (NMNH) by hydrolysis of the 5'-phosphate ester in the presence of alkaline phosphatase. The method from NMNH is time-consuming and is not cost-effective because NMNH as a precursor must be enzymatically hydrolyzed from NADH. Another method for the synthesis of NRH is the reduction of NR in the presence of sodium dithionite (Na₂S₂O₄) as a reducing agent. In this method, nicotinamide riboside triflate is reduced to NRH in an aqueous solution of sodium dithionite and potassium hydrogen phosphate (**Scheme 2**). Because the aqueous solution of Na₂S₂O₄ is very unstable at ambient conditions, this reaction must be carried out at low temperature and under anaerobic, alkaline conditions. Furthermore, the crude product should be immediately purified with HPLC using a C18 resin because NRH is sensitive to both hydrolysis and oxidation at ambient conditions as shown in the results and discussion of Example.

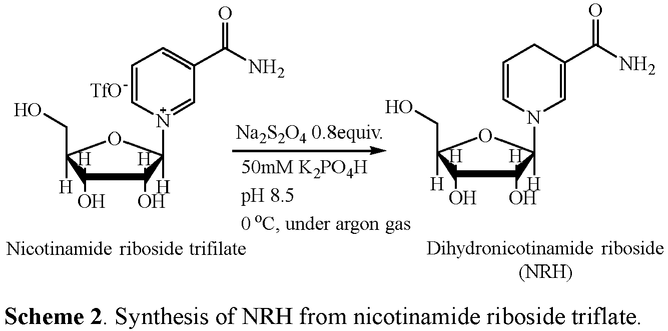

Although this method is suitable to synthesize a small amount of NRH, there are several drawbacks that prevent scaling this to the commercial production of NRH. The precursor, nicotinamide riboside triflate, is very expensive, is a very hygroscopic material, and must be stored at -20 ° C under an inert atmosphere. Moreover, this compound is not food grade because of the presence of the triflate anion in the structure of nicotinamide riboside triflate. Therefore, a complete purification of NRH from the remaining NR (triflate) is required after the reduction.

Another method for the synthesis of NRH is the use of triacetylated nicotinamide riboside triflate instead of nicotinamide riboside triflate (**Scheme 3**). In the first step of this indirect procedure, triacetylated NR converts to triacetylated NRH with Na₂S₂O₄. In the next step, the NRH is formed by methanolysis of triacetylated NRH while ball-milling. This method might be appropriate for scalable synthesis of NRH because it provides a good yield. However, the use of triacetylated nicotinamide riboside triflate, an expensive and non-food grade substance, may limit this procedure.

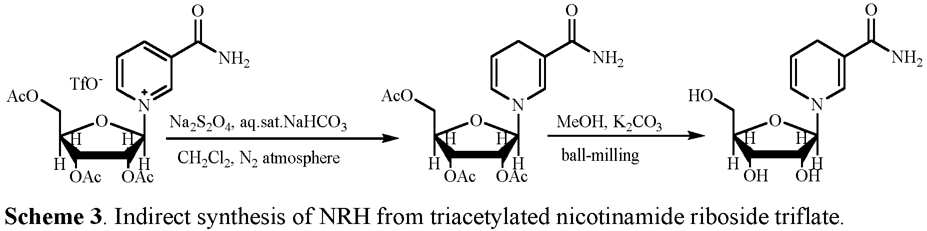

It should be noted that most of the nicotinamide ribosides that have been used for the synthesis of NRH are the mixture of two anomeric α- and β- forms. However, only the β-anomer of NR represents bioactivity and medicinal properties. Among NR derivatives, only nicotinamide riboside chloride (NRCl) is commercially available as a dietary supplement.

### SUMMARY

The present disclosure includes the recognition that using β-NRCl as a precursor to synthesize NRH would represent a breakthrough for the commercialization of the NRH, a desired and valuable product. Indeed, the method of producing NRH disclosed herein has an advantageously high yield from a commercially available NR or its derivatives such as β-NRCl.

### BRIEF DESCRIPTION OF DRAWINGS

**FIG. 1** is a diagram showing exemplary methods of producing dihydronicotinamide riboside from nicotinamide riboside chloride, according to an embodiment provided by the present disclosure;
**FIG. 2** is a set of photographs and graphs showing the TLC of synthesized NRH at pH 8.1 after purification compared to NR and NA (FIG. 2a) and FT-IR of purified NRH and pristine NR (FIG. 2b), according to an embodiment provided by the present disclosure;
**FIG. 3** is a set of graphs showing ¹HNMR of purified NRH in D₂O, according to an embodiment provided by the present disclosure;
**FIG. 4** is a set of graphs showing the ¹H NMR spectra of NRCl (FIG. 4a), NRH (FIG. 4b) and NA (FIG. 4c) in D₂O, according to an embodiment provided by the present disclosure;
**FIG. 5** is a set of photographs and graphs showing the TLC of synthesized NRH at pH 8.5 after purification compared to NRCl and NA (FIG. 5a), and the ¹HNMR of synthesized NRH at pH 8.5 after purification (FIG. 5b), according to an embodiment provided by the present disclosure;
**FIG. 6** is a set of graphs showing TGA thermograms of the NRH compared to the NRCl in the range of 25 to 900 °C under N₂ atmosphere. Weight loss vs. temperature (FIG. 6a), derivative weight loss vs. temperature (FIG. 6b), according to an embodiment provided by the present disclosure;

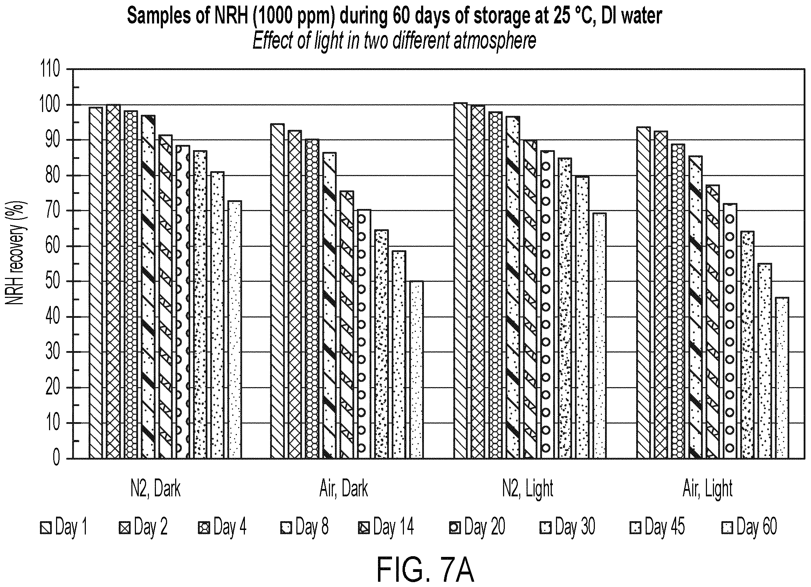

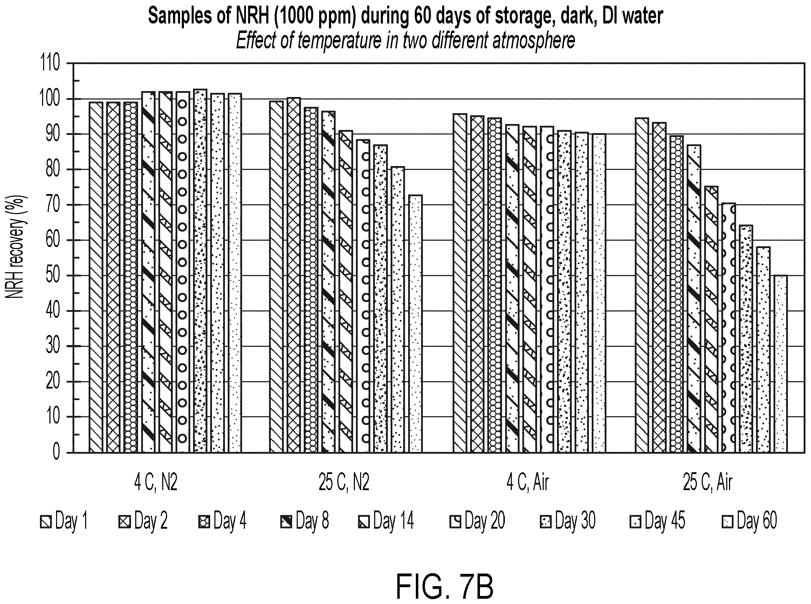

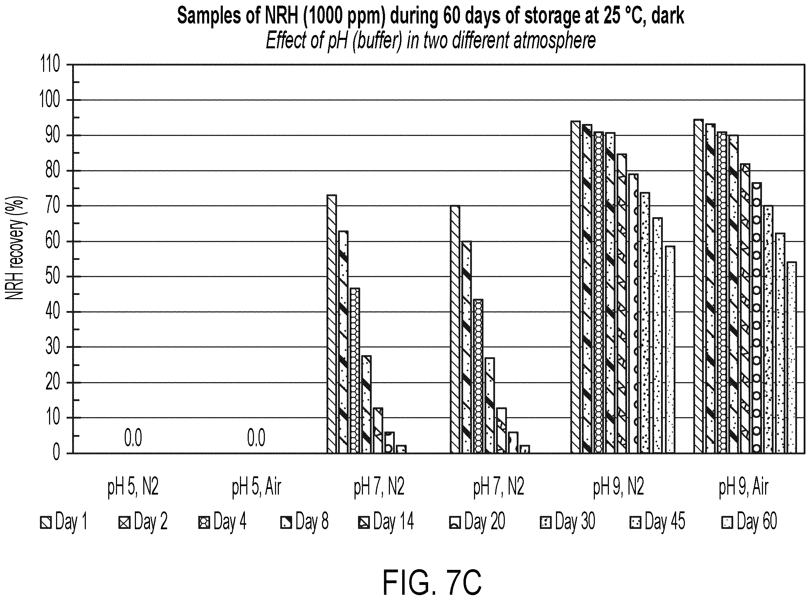

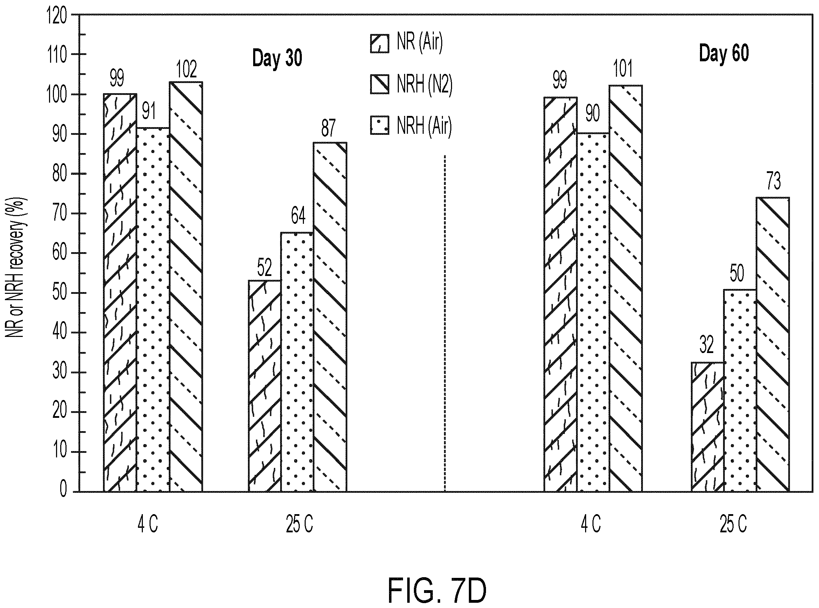

**FIG. 7** is a set of graphs showing NRH degradation kinetic study. NRH recovery (%) in aqueous samples during the 60 days of storage in the dark/light and in air/under N₂ (FIG. 7a), recovery of the NRH in aqueous solutions that are being kept at 4 °C and 25 °C during 60 days of storage in air/under N₂ (FIG. 7b), the effect of pHs 5, 7, and 9 buffers on the stability of the NRH during 60 days storage at 25 °C in air and under N₂ (FIG. 7c), NR and NRH recovery (%) in aqueous samples during the 30 days, and 60 days of storage at 4 and 25 °C (FIG. 7d), according to an embodiment provided by the present disclosure;
**FIG. 8** is a graph showing ¹H NMR Spectrum of NRH in D₂O;
**FIG. 9** is a set of graphs showing ¹H NMR Spectrum of NRH in D₂O (Expanded between 5.00-7.20 ppm);
**FIG. 10** is a set of graphs showing ¹H NMR Spectrum of NRH in D₂O (Expanded between 4.11-4.95 ppm);
**FIG. 11** is a set of graphs showing ¹H NMR Spectrum of NRH in D₂O (Expanded between 3.09-4.03 ppm);
**FIG. 12** is a graph showing ¹³C NMR Spectrum of NRH in D₂O;
**FIG. 13** is a graph showing ¹H NMR Spectrum of NRH in CD₃OD;
**FIG. 14** is a set of graphs showing ¹H NMR Spectrum of NRH in CD₃OD (Expanded between 4.76-7.22 ppm);
**FIG. 15** is a set of graphs showing ¹H NMR Spectrum of NRH in CD₃OD (Expanded between 3.06-4.12 ppm);
**FIG. 16** is a graph showing ¹³C NMR Spectrum of NRH in CD₃OD;
**FIG. 17** is a graph showing MS of NRH;
**FIG. 18** is a graph showing Selected Reaction Monitoring (SRM) of NRH;
**FIG. 19** is a graph showing UV-VIS Spectrum of NRH in H₂O;
**FIG. 20** is a set of graphs showing the HPLC chromatograms of the purified NRH **FIG. 20a**) UV detector at 220 nm, **FIG. 20b**) UV detector at 340 nm, and **FIG. 20c**) fluorescence detector;
**FIG. 21** is a set of graphs showing the HPLC chromatograms of the NRH aqueous solutions stored at different conditions for 60 days. **FIG. 21a**) at 4 °C under N2 and darkness, **FIG. 21b**) at 4 °C under air and darkness, **FIG. 21c**) at 25 °C under N2 and darkness, **FIG. 21d**) at 25 °C under air and darkness, **FIG. 21e**) at 25 °C under N2 and light, and **FIG. 21f**) at 25 °C under air and light;
**FIG. 22** is a set of graphs showing the HPLC chromatograms of the NRH aqueous solutions stored at different conditions for 60 days. **FIG. 22a**) pH= 7.0, at 25 °C, under N2 and dark, **FIG. 22b**) pH= 7.0, at 4 °C, under air and dark, **FIG. 22c**) pH= 9.0, at 25 °C, under N2 and dark, and **FIG. 22d**) pH= 9.0, at 25 °C, under air and dark; and
**FIG. 23** is a set of graphs showing Kinetic graphs and the first order degradation rates for some of NRH samples. **FIG. 23a, FIG. 23b**) pH= 7.0, at 25 °C, under N2 and air, **FIG. 23c, FIG. 23d**) pH= 9.0, at 25 °C, under N2 and air, **FIG. 23e, FIG. 23f**) DI water, at 25 °C, under N2 and air.

### DETAILED DESCRIPTION

### Definitions

Some definitions are provided hereafter. Nevertheless, definitions may be located in the "Embodiments" section below, and the above header "Definitions" does not mean that such disclosures in the "Embodiments" section are not definitions.

All percentages expressed herein are by weight of the total weight of the composition unless expressed otherwise. As used herein, "about," "approximately" and "substantially" are understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number. All numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used in this disclosure and the appended claims, the singular forms "a," "an" and "the" include plural referents unless the context clearly dictates otherwise. Thus, for example, reference to "a component" or "the component" includes two or more components.

The words "comprise," "comprises" and "comprising" are to be interpreted inclusively rather than exclusively. Likewise, the terms "include," "including" and "or" should all be construed to be inclusive, unless such a construction is clearly prohibited from the context. Nevertheless, the compositions disclosed herein may lack any element that is not specifically disclosed herein. Thus, a disclosure of an embodiment using the term "comprising" includes a disclosure of embodiments "consisting essentially of" and "consisting of" the components identified.

The term "and/or" used in the context of "X and/or Y" should be interpreted as "X," or "Y," or "X and Y." Similarly, "at least one of X or Y" should be interpreted as "X," or "Y," or "X and Y." For example, "at least one dithionite or a functionally similar reducing agent" should be interpreted as "dithionite," or "a functionally similar reducing agent," or "both dithionite and a functionally similar reducing agent."

Where used herein, the terms "example" and "such as," particularly when followed by a listing of terms, are merely exemplary and illustrative and should not be deemed to be exclusive or comprehensive. As used herein, a condition "associated with" or "linked with" another condition means the conditions occur concurrently, preferably means that the conditions are caused by the same underlying condition, and most preferably means that one of the identified conditions is caused by the other identified condition.

The term "a basic solution," as used herein, refers to a solution with a pH greater than 7.0. For example, a basic solution could have a pH in the range of about 7.5 to about 10.0, about 7.8 to about 9.0, about 7.9 to about 8.8, or preferably about 8.0 to about 8.5. When reference herein is made to the pH, values correspond to pH measured at 25 °C with standard equipment.

The term "neutral pH," as used herein, refers to a pH of about 7.0. When reference herein is made to the pH, values correspond to pH measured at 25 °C with standard equipment.

The term "room temperature," as used herein, refers to a temperature commonly used in Physics and Chemistry, namely a temperature of essentially about 293 K (or about 20°C).

The term "anaerobic condition," as used herein, refers to a condition in absence of free oxygen.

The term "aerobic condition," as used herein, refers to a condition in presence of free oxygen.

The term "dithionite," as used herein, refers to an anion of S₂O₄²⁻.

The term "nicotinamide riboside" or "NR," as used herein, refers to a form pyridine-nucleoside of vitamin B3 that functions as a precursor to nicotinamide adenine dinucleotide or NAD⁺. The Nicotinamide riboside or NR has either an α-form or a β-form. For example, the β-form of the Nicotinamide riboside or NR has a chemical structure Formula I as follows:

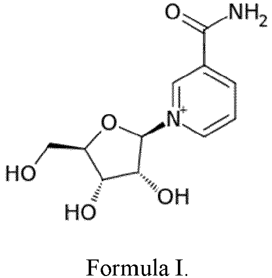

The term "alkali salt" or "basic salt," as used herein, refers to a salt that is the product of the neutralization of a strong base and a weak acid. An alkali salt or basic salt, when hydrolyzes, can form a basic solution. Examples of alkali salts or basic salts may include metal carbonates, metal bicarbonate, metal acetate, metal phosphate derivatives and others.

The term "high yield," as used herein, refers to a total yield of a synthesis at least 40%, at least 41%, at least 42%, at least 43%, at least 44%, at least 45%, at least 46%, at least 47%, at least 48%, at least 49%, at least 50%, at least 51%, at least 52%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%. Preferably, a high yield of the present disclosure is at least 50%, at least 51%, at least 52%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%.

The term "high purity," as used herein, refers to a purity of a compound or substance at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%. Preferably, a high purity of the present disclosure is at least 90%, at least 91%, at least 92%, at least 93%, at least 94%, at least 95%, at least 96%, at least 97%, at least 98%, or at least 99%.

The term "effective amount," as used herein, refers to an amount of a substance capable of either increasing (directly or indirectly) the yield of reduction product or increasing selectivity toward 1,4-dihydronicotinamide riboside (NRH). Optimum amounts of a given substance can vary based on reaction conditions and the identity of other constituents yet can be readily determined in light of the discrete circumstances of a given application.

The term "chromatography," as used herein, refers to any kind of purification technique which separates the compound of interest from other molecules in the mixture, for example, by their differences in partition between a solid substrate and the mobile phase, including but not limited to, solution, buffer or solvent and allows the compound of interested to be isolated.

The term "eluent," as used herein, refers to a solvent or a mixture of two or more solvents used in a chromatography process to move, or elute, one or more substances from a stationary support material. In an embodiment, a polar solvent such as methanol may be used as an eluent to wash out the NRH from the stationary phase during a chromatographic process.

The term "adsorbent" or "stationary phase," as used herein, refers to a material which exists in the fluid stream as a solid form in a chromatographic process. In an embodiment, a mixture of basic alumina and silica may be used as the adsorbent during a chromatographic process.

The term "solvent," as used herein, refers to a substance capable of dissolving another substance (solute).

### Embodiments

An aspect of the present disclosure is a method of producing 1,4-dihydronicotinamide riboside (NRH) from a commercial available chemical such as nicotinamide riboside chloride (NRCl). Nicotinamide riboside chloride (NRCl) is the only commercially available NR derivative (e.g., as a dietary supplement). Thus, the present method represents a new pathway for production and commercialization of 1,4-dihydronicotinamide riboside (NRH).

Applicant surprisingly found that a reduction of the commercially available nicotinamide riboside chloride (NRCl) with a reducing agent such as a dithionite in a liquid solution can lead to effective formation and production of 1,4-dihydronicotinamide riboside (NRH). Applicant further surprisingly found that the resulting 1,4-dihydronicotinamide riboside (NRH) can be isolated and purified with a high yield by using column chromatography.

In an aspect, the present disclosure relates to a method of producing 1,4-dihydronicotinamide riboside (NRH) from nicotinamide riboside chloride (NRCl).

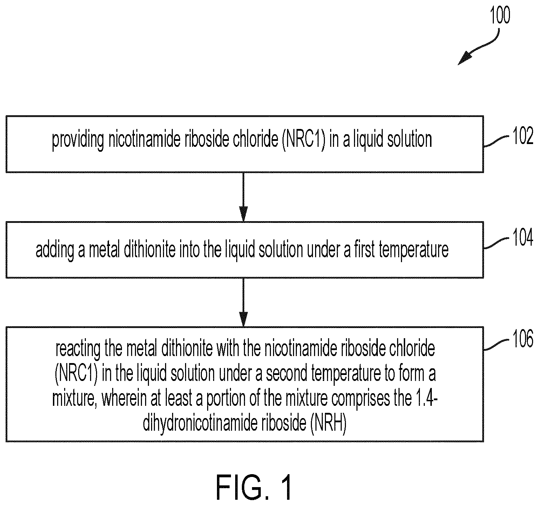

FIG. 1 shows an exemplary method (100) of producing 1,4-dihydronicotinamide riboside from nicotinamide riboside chloride, according to an embodiment provided by the present disclosure.

As shown in FIG. 1, the method 100 comprises providing nicotinamide riboside chloride (NRCl) in a liquid solution (102).

Nicotinamide riboside chloride (NRCl) is commercially available, and could be purchased as, e.g., Niagen^{™} from ChromaDex.

Nicotinamide riboside chloride (NRCl) includes either α-form NRCl (α-NRCl) or β-form NRCl (β-NRCl). In an embodiment, the nicotinamide riboside chloride (NRCl) of present disclosure comprises β-NRCl. In an embodiment, the nicotinamide riboside chloride (NRCl) of present disclosure is β-NRCl.

In an embodiment, as only the β-anomer of NR represents bioactivity and medicinal properties, the present disclosure uses β-NRCl as the exemplary starting material to produce 1,4-dihydronicotinamide riboside (NRH). The present method of producing 1,4-dihydronicotinamide riboside (NRH) can also be applied to using α-NRCl as a starting material.

A chemically effective amount of nicotinamide riboside chloride (β-NRCl) as a form of solid (e.g., a powder) is provided. In an embodiment, the solid form of nicotinamide riboside chloride (β-NRCl) may be used in a solid-state reaction to produce 1,4-dihydronicotinamide riboside (NRH). An exemplary solid-state reaction was described in the Example.

In another embodiment, the solid form of β-nicotinamide riboside chloride (β-NRCl) is dissolved in a solvent such as water to form a liquid solution for the production of 1,4-dihydronicotinamide riboside (NRH).

In a preferred embodiment, a chemically effective amount of β-nicotinamide riboside chloride (β-NRCl) is first dissolved in a solution such as water to form a solution for a liquid-based production of 1,4-dihydronicotinamide riboside (NRH).

As shown in FIG. 1, after a chemically effective amount of β-nicotinamide riboside chloride (β-NRCl) is provided in a liquid solution, the method 100 may comprise adding a metal dithionite into the liquid solution under a first temperature (104). As such, a reducing agent such as a metal dithionite may be added into the liquid solution.

In an embodiment, the reducing agent may comprise a metal dithionite such as sodium dithionite (Na₂S₂O₄). In an embodiment, the only reducing agent may be a metal dithionite such as sodium dithionite (Na₂S₂O₄). Scheme 4 shows an exemplary reduction reaction of β-nicotinamide riboside chloride (β-NRCl) with sodium dithionite (Na₂S₂O₄) in a liquid solution.

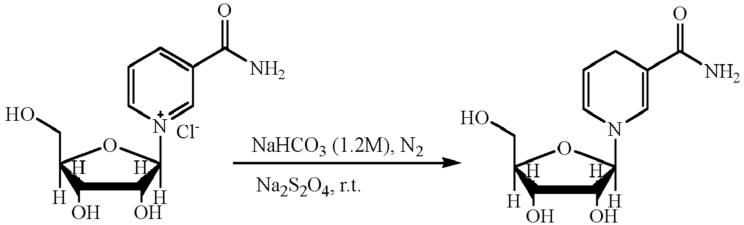

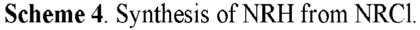

Scheme 5 shows a reaction mechanism of NRH synthesis from NR by using Na₂S₂O₄ as a reducing agent. As shown in Scheme 5, a reduction reaction of NR by using Na₂S₂O₄ initially forms a sulfinate intermediate, which is stable at basic conditions.

After protonation, the sulfinate intermediate forms its sulfinic acid derivative. This sulfinic acid intermediate is unstable at ambient conditions and converts to NRH via SO₂ leaving.

As such, applicant notes that a particularly preferred embodiment uses a pH that will not only stabilize Na₂S₂O₄ but will also protonate the sulfonate intermediate to produce the NRH. Moreover, because NRH has an N-glycoside bond in its structure, NRH is susceptible to hydrolysis. Consequently, a particularly preferred embodiment adjusts the pH and maintains the pH precisely during the whole course of the reaction so that NRH is not hydrolyzed.

Thus, the present methods include adjusting the pH of the related solutions and maintaining it precisely within a specific range (e.g., 8.0-8.5, preferably 8.1) for effective production of NRH.

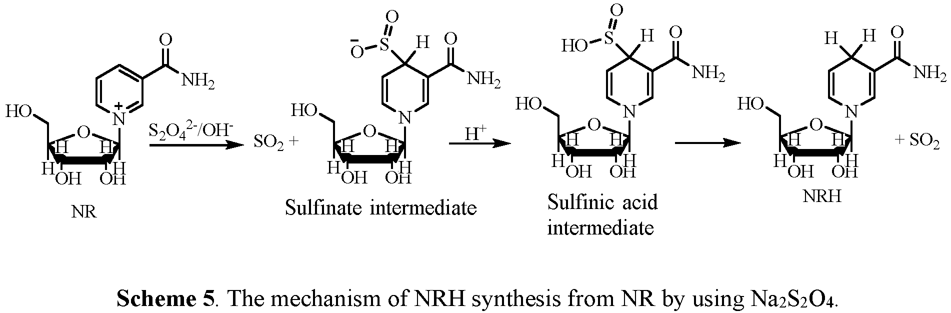

Throughout this disclosure, Na₂S₂O₄ was used as an exemplary reducing agent. Other functionally similar reducing agent may also be used. Preferably, the reducing agent is Na₂S₂O₄.

A molar ratio of β-nicotinamide riboside chloride (β-NRCl) to the reducing agent such as Na₂S₂O₄ is preferably between about 1:1 and about 1:5, between about 1:1.5 and about 1:4, or between about 1:2 and about 1:3, preferably between about 1:2 and about 1:3, more preferably about 1:2.7.

As an aqueous solution of Na₂S₂O₄ is unstable under aerobic conditions at neutral pH (e.g., pH=7), preferably the liquid solution for the reduction reaction of β-nicotinamide riboside chloride (β-NRCl) by using Na₂S₂O₄ is maintained under anaerobic and basic conditions.

For example, the liquid solution may be purged with an inert gas to remove free oxygen in the solution. An inert gas may include nitrogen (N₂), argon (Ar) or others. In an embodiment, either nitrogen or argon is used as the inert gas. The liquid solution may be purged with an inert gas throughout the reduction reaction.

In an embodiment, the inert gas comprises nitrogen gas. In an embodiment, the only inert gas is nitrogen gas.

In an embodiment, the liquid solution comprises a basic solution. In an embodiment, the liquid solution is a basic solution.

In an embodiment, the basic solution comprises sodium bicarbonate (NaHCO₃). For example, the liquid solution may include at least one alkali salt or basic salt such as sodium bicarbonate (NaHCO₃) to form a basic solution. In an embodiment, the only alkali salt or basic salt is sodium bicarbonate (NaHCO₃). Nevertheless, other alkali salt or basic salt such as sodium carbonate, potassium carbonate, potassium bicarbonate and similar compounds may optionally be used.

In an embodiment, a pH value of the liquid solution may be controlled by the concentration of the at least one alkali salt or basic salt in the solution. For example, by using 1-1.5 M, preferably 1.1-1.4M, more preferably 1.2M of NaHCO₃, the pH value of the liquid solution may be controlled in the range of about 8.0 to about 8.5, about 8.05 to about 8.45, about 8.07 to about 8.35, about 8.08 to about 8.29, about 8.09 to about 8.19, preferably about 8.1.

In an embodiment, the method 100 comprises maintaining a pH value of the NaHCO₃ solution between 8.0-8.5. In an embodiment, the method 100 comprises maintaining a pH value of the NaHCO₃ solution between about 8.0 and about 8.5. In an embodiment, the method 100 comprises maintaining a pH value of the NaHCO₃ solution about 8.1.

In an embodiment, the NaHCO₃ solution has a concentration from about 1.0 to about 1.5 M. In an embodiment, the NaHCO₃ solution has a concentration about 1.2 M.

In an embodiment, the reducing agent such as Na₂S₂O₄ may be gradually added into the liquid solution of the β-nicotinamide riboside chloride (β-NRCl) under an inert gas atmosphere at a first temperature. In an embodiment, the reducing agent such as Na₂S₂O₄ may be pre-dissolved into a solvent such as water and the solution of the reducing agent such as Na₂S₂O₄ may be dropped-wise into the liquid solution of the β-nicotinamide riboside chloride (β-NRCl) under an inert gas atmosphere at a first temperature.

In an embodiment, the first temperature is a low temperature such as about 0°C (e.g., by using ice water to control) so that the reduction reaction can be controlled during the addition of the reducing agent such as Na₂S₂O₄.

Returning to FIG. 1, after the addition of the reducing agent such as Na₂S₂O₄, the method 100 may comprise reacting the metal dithionite with the nicotinamide riboside chloride (NRCl) in the liquid solution under a second temperature to form a mixture, wherein a portion of the mixture is the 1,4-dihydronicotinamide riboside (NRH) (106).

As such, the temperature of the liquid solution is preferably increased from the first temperature to a second temperature. Under the second temperature, the nicotinamide riboside chloride (NRCl) in the liquid solution can completely and effectively react with the reducing agent such as Na₂S₂O₄ to form the 1,4-dihydronicotinamide riboside (NRH). In an embodiment, the second temperature is room temperature.

Under the room temperature, the reducing agent such as Na₂S₂O₄ and the β-nicotinamide riboside chloride (β-NRCl) may continuously and completely react to form a mixture, wherein at least portion of the mixture is the 1,4-dihydronicotinamide riboside (NRH).

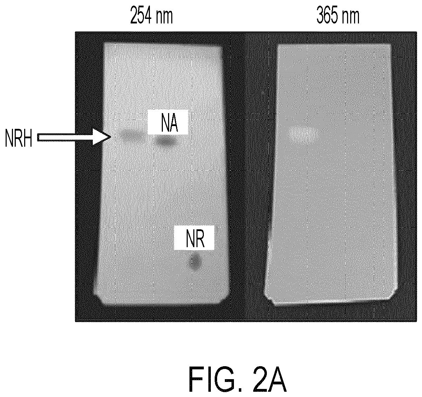

In an embodiment, progress of the reduction reaction between the reducing agent such as Na₂S₂O₄ and the β-nicotinamide riboside chloride (β-NRCl) may be monitored by using a chromatography technique. For example, as shown in FIG. 2a, all compounds of β-NRCl, NRH and nicotinamide (NA; a degradation product from NRH; see Scheme 6) were visible at a wavelength of 254 nm. However, at a wavelength of 365 nm, only NRH was visible. By monitoring intensity and position of peaks corresponding to starting material β-NRCl and the resulting product NRH, one can determine progress of the reduction reaction between the reducing agent such as Na₂S₂O₄ and the β-nicotinamide riboside chloride (β-NRCl).

After the reduction reaction between the reducing agent such as Na₂S₂O₄ and the β-nicotinamide riboside chloride (β-NRCl) is complete, the resulting mixture may be dried to obtain a solid of the mixture. In an embodiment, the resulting mixture may be freeze-dried to obtain a yellow solid of the mixture.

In another aspect of the present disclosure, a purification method for obtaining a high purity of the resulting NRH is disclosed. Applicant surprisingly found that the resulting 1,4-dihydronicotinamide riboside (NRH) can be isolated and purified with a high yield by using column chromatography. Examples below provide a detailed exemplary purification method by using column chromatography (e.g., with a mixture of basic alumina and silica as the adsorbent).

In an embodiment, a chromatography method may be used to isolate the resulting NRH from the mixture. For example, column chromatography can be used to separate NRH from β-NRCI or other impurity substances based on differential adsorption of compounds to the adsorbent; compounds move through the column at different rates, allowing them to be separated into fractions.

In an embodiment, the column chromatograph method uses an alcohol as its eluent. In an embodiment, the eluent comprises methanol. In an embodiment, the only eluent is methanol. Nevertheless, other solvents such as a mixture of methanol and ethanol can optionally be used as eluent additionally or alternatively.

In an embodiment, the adsorbent of the column chromatograph comprises at least basic alumina and silica. In an embodiment, basic alumina and silica are the only adsorbents of the column chromatograph.

The basic alumina and the silica may have a weight ratio of between about 1:10 and about 10:1, between about 1:9 and about 9: 1, between about 1:8 and about 8:1, between about 1:7 and about 7:1, between about 1:6 and about 6:1, between about 1:5 and about 5:1, between about 1:4 and about 4:1, between about 1:3 and about 3:1, or between about 1:2 and about 2:1, preferably about 2:3.

In an embodiment, a column chromatograph purification method may use basic alumina with silica, for example with the weight ratio of about 2:3, to isolate NRH with a purity at least 70%, at least 72%, at least 75%, at least 77%, at least 80%, at least 83%, at least 85%, at least 87%, at least 90%, at least 93%, at least 95%, at least 96%, or at least 98%, preferably at least 96%.

In an embodiment, the column chromatograph purification method by using basic alumina with silica may remove at least 90%, at least 95%, at least 99%, at least 99.9%, or at least 99.999 of the unreacted NRCl in the mixture. In a preferred embodiment, the column chromatograph purification method by using basic alumina with silica may completely purify the NRH product from the unreacted NRCl in the mixture.

In an embodiment, as demonstrated in Examples, applicant's results show that column chromatography on either silica or basic alumina alone was not effective in separating NR from NRH. Thus, a mixture of basic alumina and silica may be the necessary adsorbent of the column chromatograph.

Basic alumina, as a polar surface with many hydroxyl groups on it, can presumably physically separate NR from NRH to a moderate degree (by difference of their polarities). Moreover, because basic alumina has negative charges on its surface and NR has a positive charge, basic alumina could act as a cationic exchange resin to immobilize NR on the surface of the basic alumina.

One may believe this is the main reason why alumina is a better stationary phase compared to silica for the purification of the NRH. However, Applicant discovered that during the purification of NRH in the column chromatography, basic alumina would clog and result in a very slow flow rate that led to the degradation of NRH.

In an embodiment, the total yield of NRH for the present method may be at least 35%, at least 40%, at least 45%, at least 50%, at least 55%, at least 60%, at least 65%, at least 70%, at least 75%, at least 80%, at least 85%, or at least 90%, preferably at least 55%.

In an embodiment, the present method of producing NRH in a liquid solution produces a significantly higher yield than that of a solid-state method. For example, the present method of producing NRH in a liquid solution produces a yield at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 120%, at least 140%, at least 160%, at least 180%, at least 200%, at least 220%, at least 240%, at least 260%, at least 280%, at least 300%, at least 320%, at least 340%, at least 360%, at least 380%, at least 400%, at least 420%, at least 440%, at least 460%, at least 480%, at least 500%, or at least 600% higher than that of a solid-state method.

Examples below provide a detailed exemplary solid-state method of producing NRH with a yield between about 15% and about 17%.

As shown in the Examples, the exemplary solution-based methods of producing and purifying NRH, according to certain embodiments of the present disclosure, demonstrate a yield in the range of about 42% to about 55%.

Thus, the present method of producing NRH in a liquid solution may produce a yield at least 267% higher than that of the exemplary solid-state method.

In another aspect, the present disclosure relates to stabilities of the NRH as produced from methods as discussed herein.

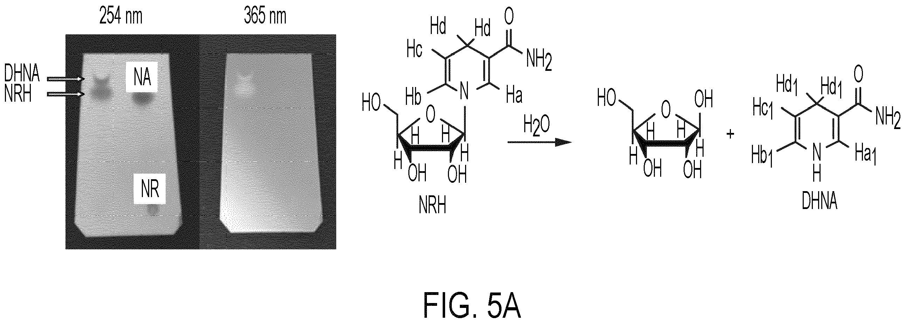

In an embodiment, a certain percentage of the NRH as produced would hydrolyze to form 1,4-dihydronicotinamide (DHNA). As shown in the Scheme of FIG. 5a, 1,4-dihydronicotinamide (DHNA) was likely formed by hydrolysis of the NRH during the course of the reaction (e.g., pH = 8.5).

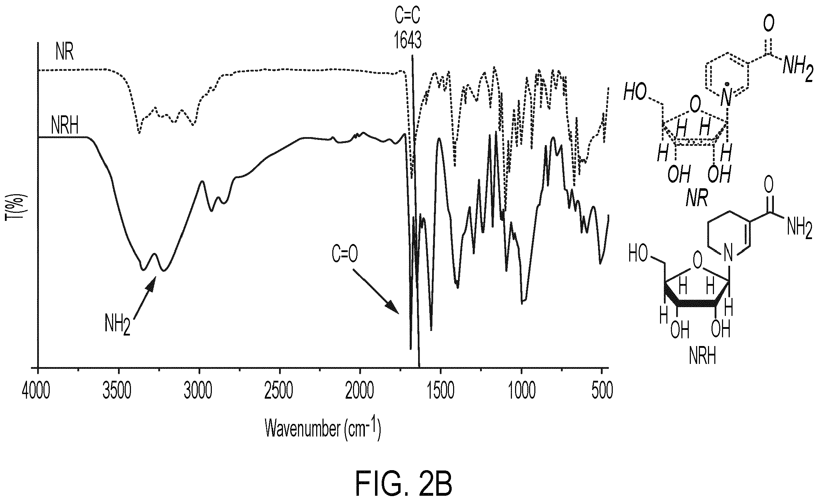

As shown in FIG. 2, when pH=8.1, there were only trace peaks, corresponding to NA in the ¹HNMR of purified NRH. This signifies that only a trace amount of NR hydrolyzes during the course of the reaction to form NA (See Scheme 6).

In an embodiment, a pH value of the liquid solution of the method of producing NRH may be maneuvered to control a reaction rate of the method and hydrolysis of both the starting material of NRCl and the product of NRH.

For example, by increasing the pH from 8.1 to 8.5, the reaction rate obviously decreases and, subsequently, there is an increased possibility of side reactions, such as hydrolysis of both NR and NRH.

In an embodiment, a pH value about 8.1 may be preferred for increasing the reaction rate of the reduction, while decreasing possibility of side reactions, such as hydrolysis of both NR and NRH.

In an embodiment, the pH of the reaction for producing NRH may be necessarily adjusted to minimize the formation of NA and DHNA to decreasing the reaction time. For example, the retardation factor (Rf) values of these by-products of NA and DHNA are close to the R_{f} values of NRH. Therefore, NA and DHNA cannot be separated from the NRH product by common column chromatography (see FIG. 5a). In a preferred embodiment, the pH of the reaction for producing NRH may be adjusted to about 8.1.

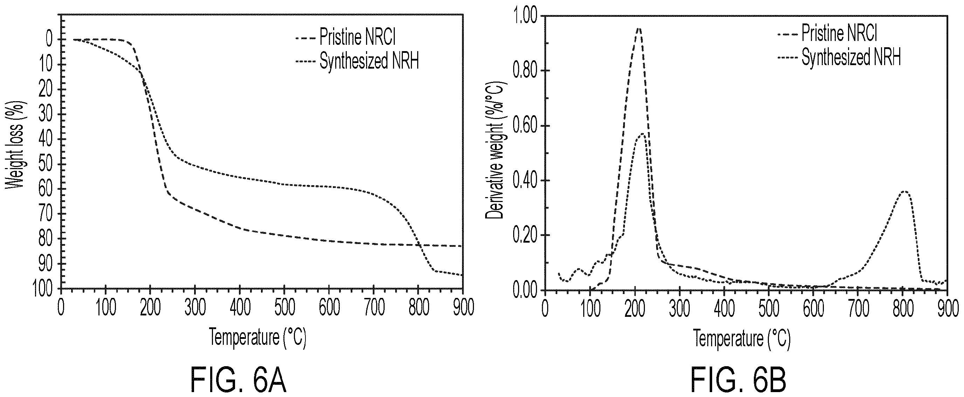

In an embodiment, the NRH is more thermally stable than the NRCl under a nitrogen atmosphere. FIGs. 6a and 6b showed that while both NRH and NR show their greatest weight loss at around 218 °C and 910 °C, respectively, NRH shows only around 50% weight loss at this temperature, while NRCl shows around 65% weight loss at this temperature. NRH shows another distinctive weight loss with a peak at around 800 °C which contributes to 30% weight loss.

In an embodiment, NRH may be susceptible to oxidation in the presence of air and NRH may be more stable under a N₂ atmosphere than that in the presence of air, as demonstrated in FIG. 7. Further, pure NRH (as a powder) may be stable in a refrigerator, in a sealed tube, for a few months without any degradation.

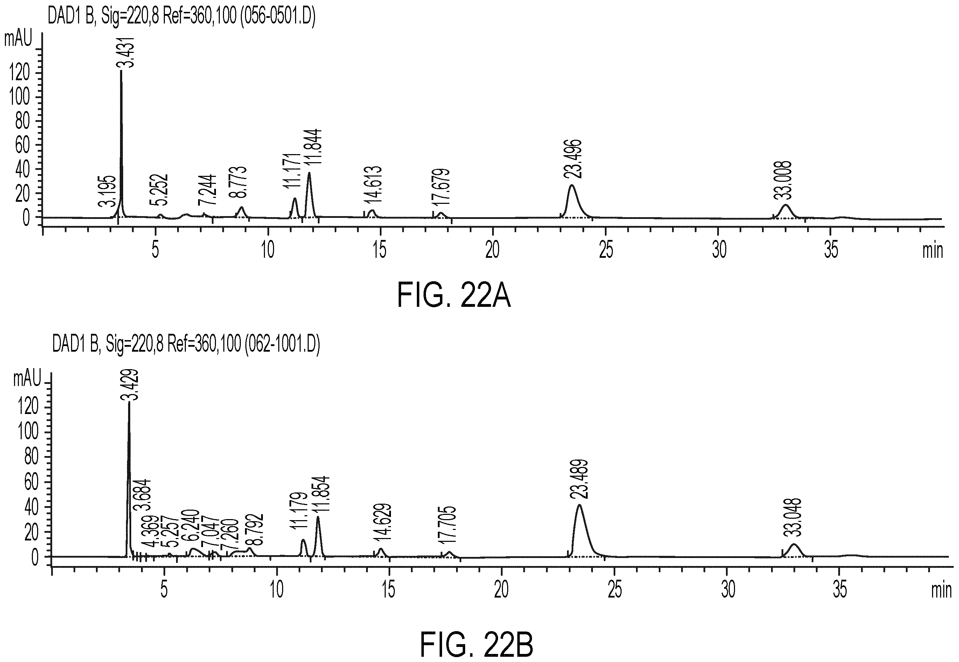

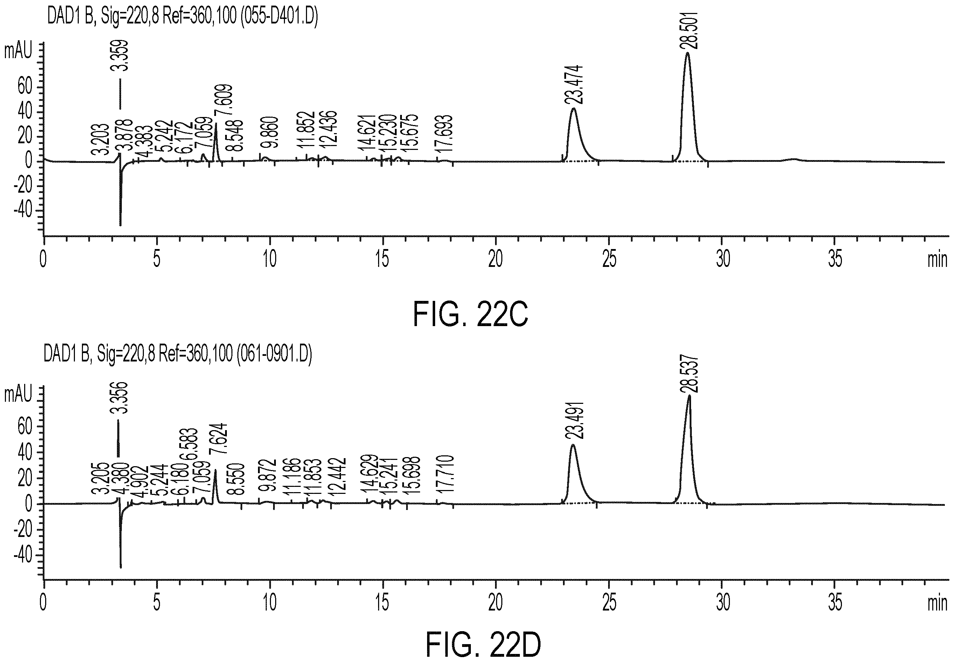

In an embodiment, as demonstrated in FIG. 7c and FIG. 22, the NRH product may be completely degraded in less than one day at pH 5, while the NRH products prepared in the ammonium acetate buffer at pH 7 may show a linear decrease of NRH concentration from around 70% in day one to around 2% in day 30.

Further, the NRH products prepared in the carbonate buffer at pH 9 may be air stable both in air (e.g., around 45% degradation measured after 60 days) and under N₂ (e.g., around 42% degradation measured after 60 days).

In an embodiment, the NR in an aqueous solution may be very stable with no detectable degradation after 60 days, while NRH in an aqueous solution stored in air may show 9% and 10% degradation after 30 and 60 days, respectively.

In an embodiment, NRH in an aqueous solution stored under a N₂ blanket may be fairly stable with no detectable degradation after 60 days of storage.

### EXAMPLE

The following non-limiting example presents scientific data developing and supporting the concept of methods and processes for producing 1,4-dihydronicotinamide riboside from nicotinamide riboside chloride and related purification and stability studies.

In the present work, we describe an efficient method for synthesis and purification of 1,4-dihydronicotinamide riboside (NRH) from commercially available nicotinamide riboside chloride (NRCl) and in the presence of sodium dithionite as a reducing agent. The method is potentially scalable. NRH is industrially relevant as the most effective, synthetic NAD⁺ precursor.

We demonstrated that solid phase synthesis cannot be used for the reduction of NRCl to NRH in high yield, whereas a reduction reaction in water at room temperature under anaerobic conditions is shown to be very effective, reaching a 55% isolation yield. For the first time, by using common column chromatography, we were able to highly purify this sensitive bio-compound with good yield. A series of identifications and analyses including HPLC, NMR, LC-MS, FTIR and UV-Vis spectroscopy, were performed on the purified sample, confirming the structure of NRH as well as its purity to be 96%. Thermal analysis of NRH showed higher thermal stability compared to NRCl, and with two major weight losses, one at 218 °C and another at 805 °C. We also investigated the long term stability effects of temperature, pH, light, and oxygen (as air) on the NRH in aqueous solutions. Our results show that NRH can be oxidized in the presence of oxygen, and it hydrolyzed quickly in acidic conditions. We also found that the degradation rate is lower under N₂ atmosphere, at lower temperatures, and in basic pHs.

### Experimental section

**Materials.** NR chloride (beta form) was a gift from ChromaDex Company. Sodium dithionite was purchased from VWR, sodium hydrogen carbonate was purchased from Aldrich, silica gel (P60, 40-63 µm, 60 Å) was purchased from SiliCycle, and basic alumina (50-200 µm, 60 Å, pH 8) was purchased from Acros. Methanol (99.9%, Certified ACS, Fisher), acetone (99.8% Certified ACS, Fisher), sodium hydroxide (certified ACS, Fisher Chemical), Hexanes (≥98.5%, GR ACS), ethyl acetate (EtOAc, >99.9%, certified ACS) and Silica Gel 60 F254 Coated Aluminum-Backed TLC Sheets were purchased from EMD Millipore (Billerica, MA, USA). Deuterated water and dimethyl sulfoxide (DMSO-d6, D, 99.9%) were purchased from Cambridge Isotope Laboratories, Inc.

**Characterization.** A 500 NMR (Bruker INOVA) spectrometer was used to prepare the ¹H and ¹³C-NMR spectra in deuterated water. Fourier transform infrared spectra (FTIR) were recorded on a Shimadzu IRAffinity-1S spectrophotometer by collecting 128 scans with a resolution of 8 cm⁻¹. UV-vis spectra of the NR and NRH solutions were recorded on a Shimadzu UV-2600 spectrophotometer. Thermogravimetric analysis (TGA) thermograms were prepared in the range of 20-900 °C at a temperature rate of 10°C min⁻¹ under N₂ flow, using a TA Q100 instrument. An Agilent 1200 LC System equipped with Binary SL Pump & Diode Array Detector and a Shodex RI-501 Refractive Index Detector (single channel) was used to perform the high-performance liquid chromatography (HPLC) measurements. Reversed-phase HPLC was performed on a Discovery C18 Column, 180 Å (pore size), 5 µm diameter, 250×4.6 mm in dimension. Ammonium acetate (20 mM) was used as the mobile phase with a flow rate of 1.0 mL min⁻¹ over 30 or 45 min at 25 °C. All samples were filtrated using a 13 mm Nylon syringe filter with a 0.22 µm pore size before measurement. For LC-MS analysis, we used LC (Agilent 1100 series) coupled with a mass spectrometer. Reverse-phase chromatography was used with a Phenomenex Luna Omega (Phenomenex) LC column with the following specifications: 100 × 4.6 mm, 3 µm, polar C18, 100 Å pore size with a flow rate of 0.3 mL min⁻¹. LC eluents include ammonium acetate 20 mM (solution A) and acetonitrile (solution B) using gradient elution (solution A:B composition change with time: 0 min: 95:5, 3 min: 95:5, 15 min: 85:15, 17 min: 90:10, and 20 min 95:5). The mass spectrometer (Finnigan LTQ mass spectrometer) was equipped with an electrospray interface (ESI) set in positive electrospray ionization mode for analyzing the NRH. The optimized parameters were a sheath gas flow rate at 20 arbitrary unit, spray voltage set at 4.00 kV, capillary temperature at 350 °C, capillary voltage at 41.0 V, and tube lens set at 125.0 V.

### Synthesis of 1,4-dihydronicotinamide riboside from β-nicotinamide riboside chloride

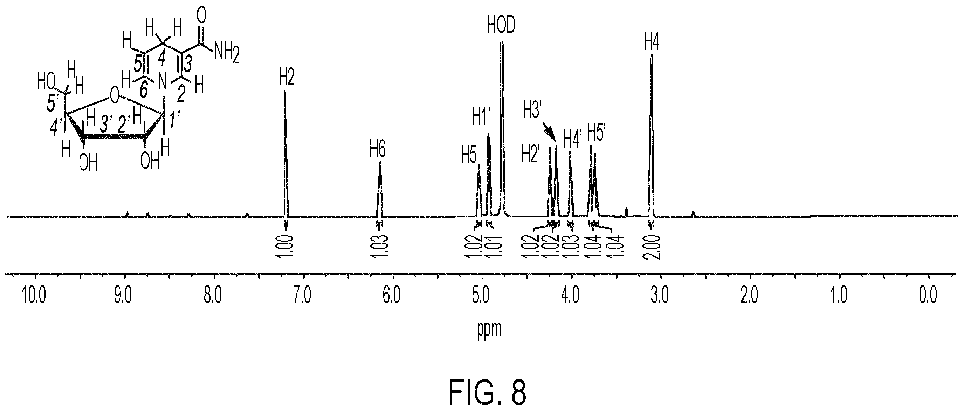

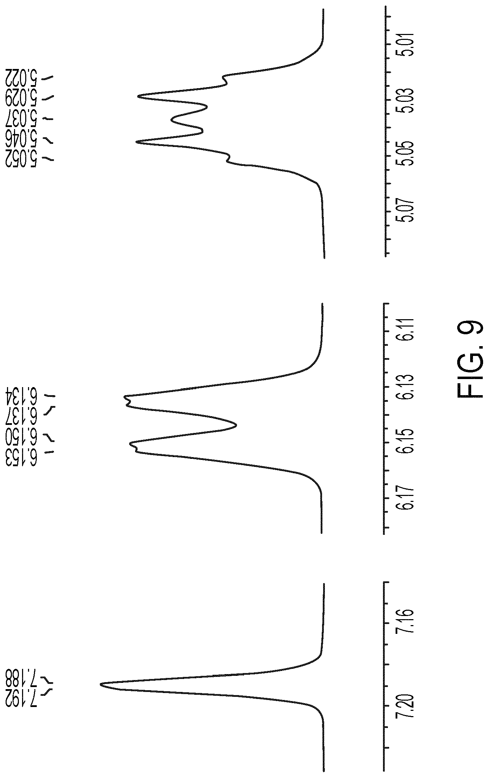

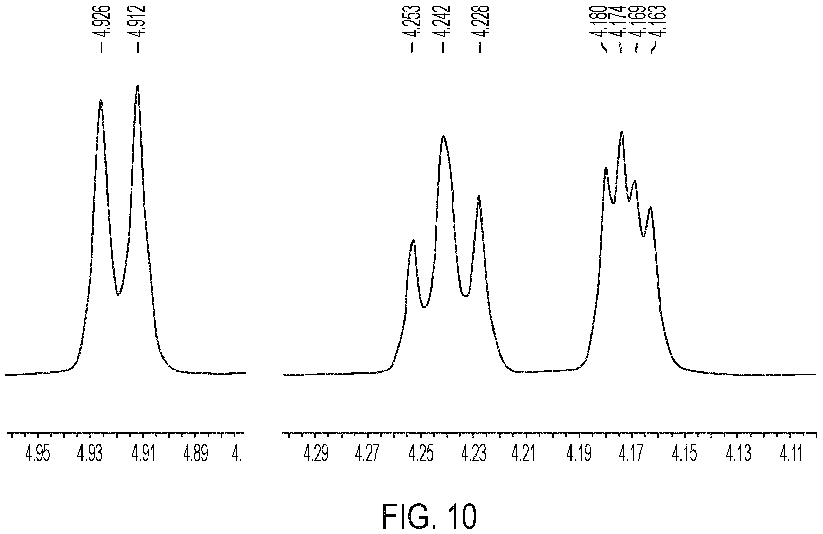

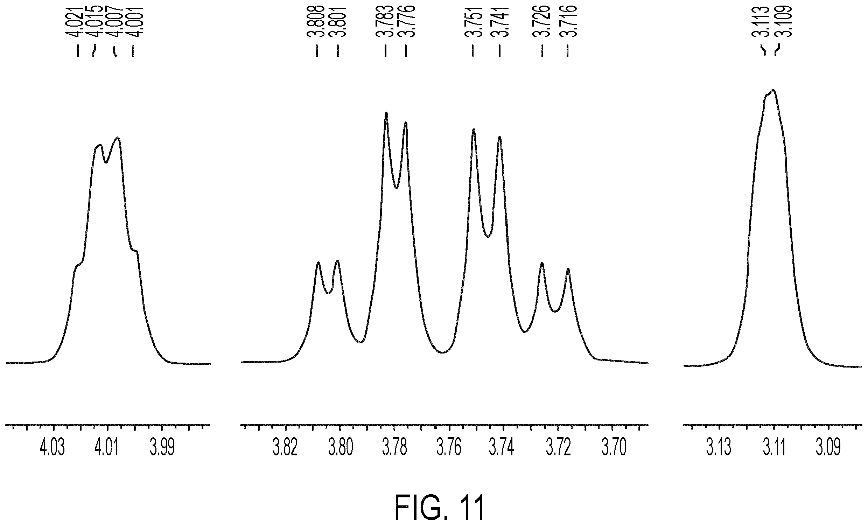

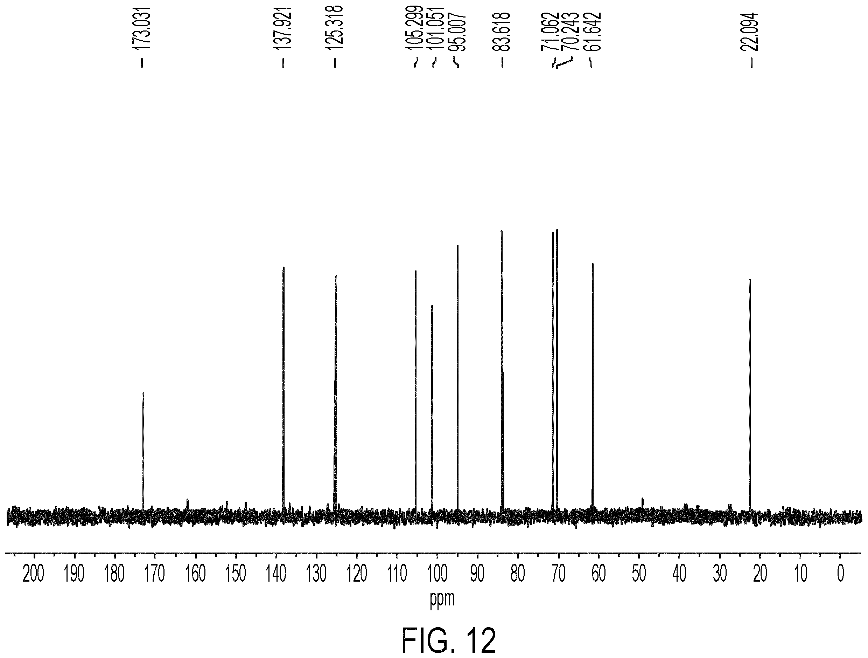

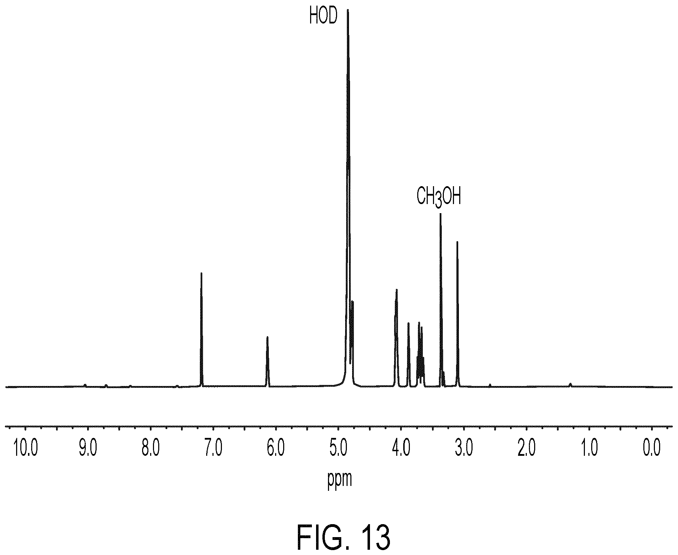

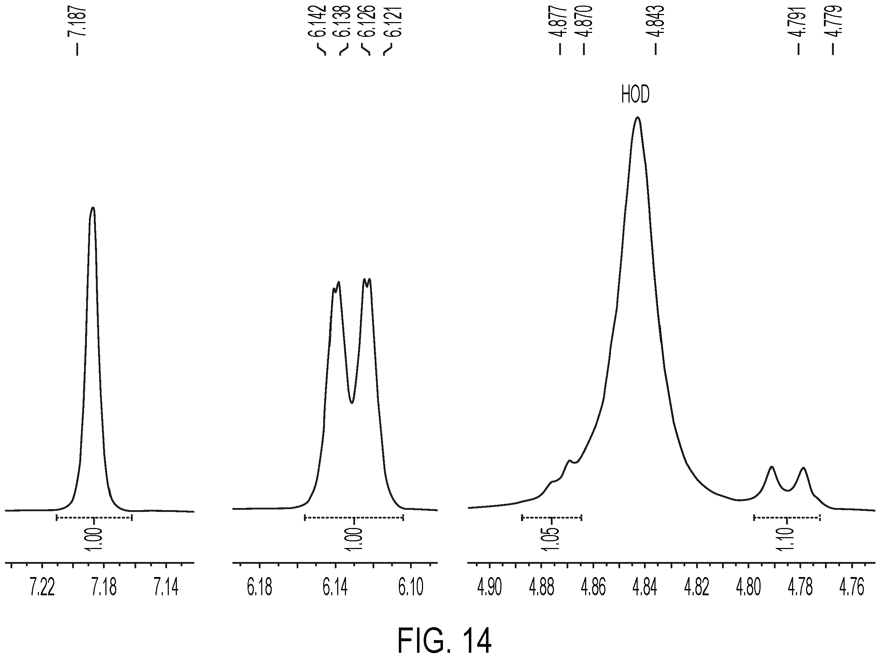

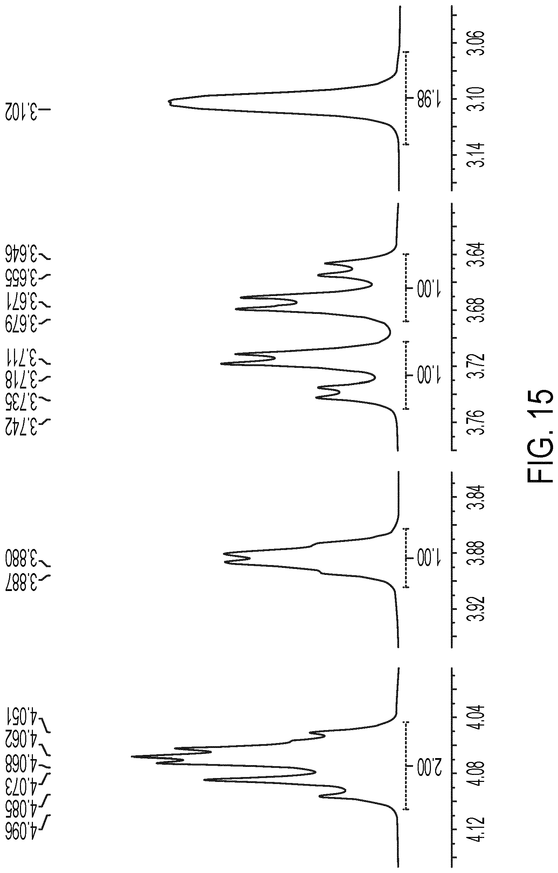

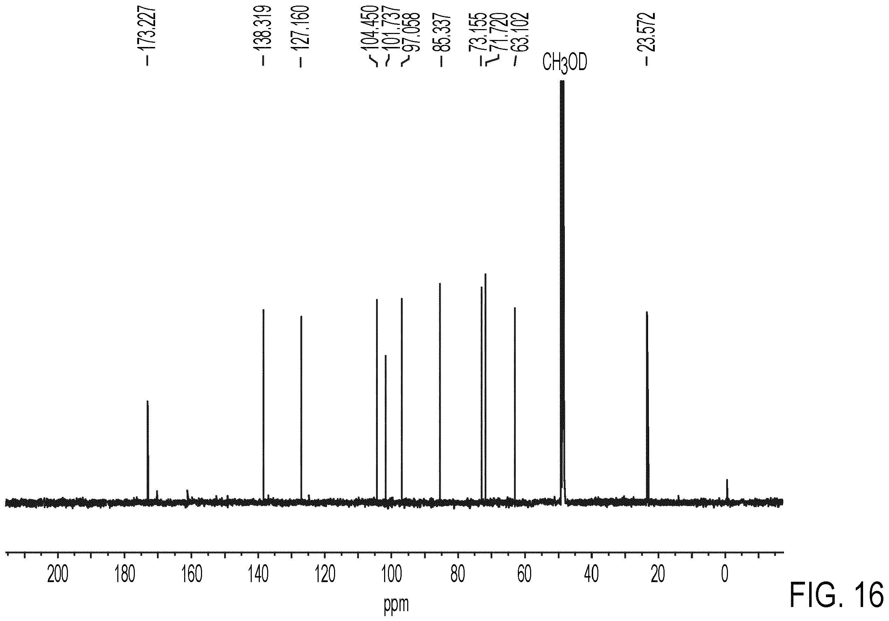

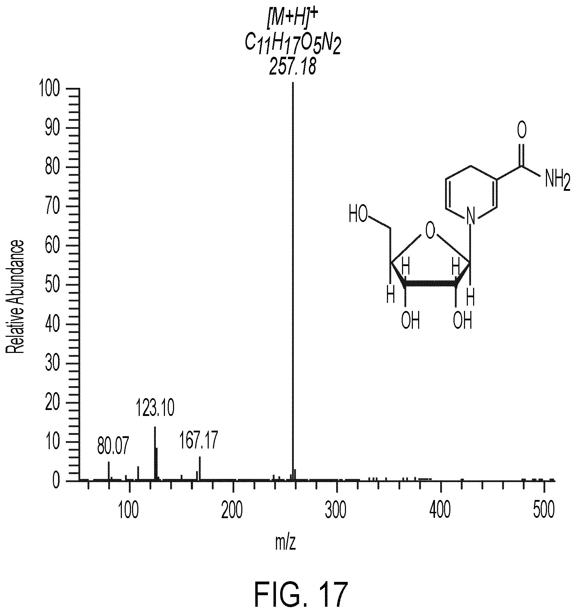

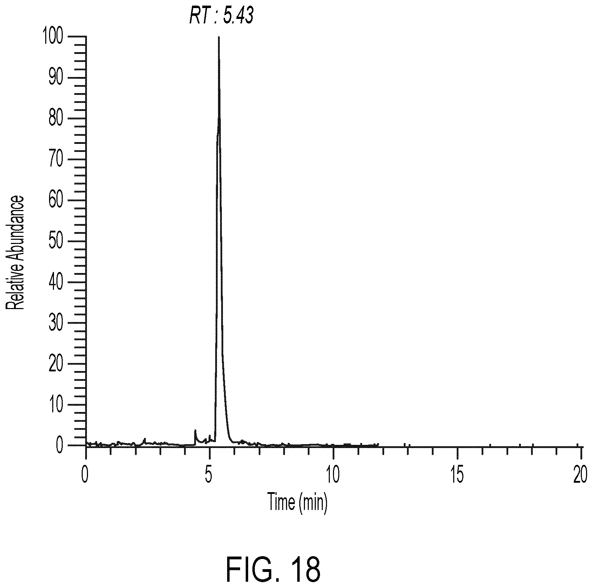

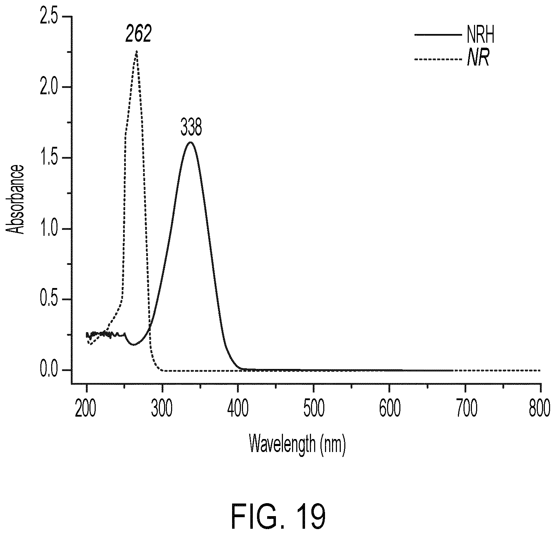

0.5 g of NRCI (1.72 mmol) and 20 mL of NaHCO₃ solution (1.2 M) were added to a round bottom flask with a magnetic stir bar. This system was placed in an ice bath, purged of oxygen, and kept under nitrogen gas. Then, 0.80 g of sodium dithionite (4.60 mmol) was gradually added to the reaction mixture. After adding Na₂S₂O₄, the flask was taken out of ice bath, and subsequently the reaction was carried out at room temperature for three extra hours. The reaction mixture was freeze-dried to obtain a yellow solid. Finally, the residue was purified by column chromatography using a mixture of basic alumina and silica with the weight ratio of 2:3 respectively, using methanol as eluent. The extra methanol was removed by rotary evaporator at room temperature to obtain a pale yellow, sticky solid that was next converted to a yellow powder (precipitate) by adding ethyl acetate. Finally, the isolated product was washed with n-hexane and dried under reduced pressure at room temperature to obtain pure NRH in 55% yield (0.24 g). ¹H NMR (500 MHz, D₂O), δ ppm: 7.19 (s, 1H), 6.14 (dd, *J₁* = 8.2 Hz, *J₂*= 1.5 Hz, 1H), 5.05-5.02 (m, 1H), 4.92 (d, *J*= 7 Hz, 1H), 4.24 (t, *J*= 5.5 Hz, 1H), 4.18-4.16 (m, 1H), 4.03-3.99 (m, 1H), 3.79 (dd, *J₁*= 12.5 Hz, *J₂*= 3.5 Hz, 1H), 3.73 (dd, *J₁*= 12.5 Hz, *J₂*= 5.0 Hz, 1H), 3.11 (s, 2H) (FIGs. 8-11). ¹³C NMR (125 MHz, D₂O), δ ppm: 173.03, 137.92, 125.32, 105.30, 101.05, 95.01, 83.62, 71.06, 70.24, 61.64, 22.09 (FIG. 12). In addition, ¹HNMR and ¹³C NMR spectra of purified NRH in CD₃OD visuals are in the supporting information section (FIGs. 13-16) where MS: found m/z = 257.18 (M+1). Calculated for C₁₁H₁₇N₂O₅ (M+1): 257.11 (FIGs. 17 and 18). UV (λₘₐₓ in H₂O): 338 nm (FIG. 19).

### Solid-state synthesis of NRH from NRCl

In the first step, to decrease the hydrolysis of NRH during the course of the reaction, we set up the reduction reaction in a solvent-free environment. We used SiO₂ as a solid support to increase the surface on which the reaction occurs. In this procedure, SiO₂ (0.25 g), NRCl (0.1 g, 0.34 mmol), Na₂S₂O₄ (0.2 g, 1.15 mmol), and NaHCO₃ (0.25 g) were placed in a mortar and ground for 5 minutes to obtain a homogeneous powder. Then, 1 mL of DI water was dropwise added to the reaction mixture and the reaction was ground for 10 minutes at room temperature (**Table 1, entry 1**). It should be mentioned that without using the solid support the reaction mixture becomes sticky and is not easily ground. After grinding, the products were extracted by MeOH and the progress of the reaction was followed by TLC (thin layer chromatography). The results showed a trace amount of NRH to be the one product that we could not isolate and purify for further identification. We hypothesized that, in the absence of enough water, the sulfinate intermediate cannot be protonated to produce sulfinic acid intermediate and then NRH as the product (**Scheme 5**). To test this hypothesis, we repeated this procedure with an alternate final step. At the end, we transferred the reaction mixture to a round bottom flask with a magnetic stir bar, added 8 mL of NaHCO₃ solution (1 M), and let the reaction continue on for 30 min at room temperature. Then, the reaction mixture was freeze-dried and the residue was purified by column chromatography in a mixture of basic alumina and silica, using methanol as the eluent. The isolated NRH was obtained with 15% yield (**Table 1, entry 3**). By changing the solid support (Al₂O₃ instead of SiO₂), no remarkable improvement was observed in the yield of the product (**Table 1, entries 2, 4**).

The ability to work under ambient conditions is an advantage of this procedure, but low yield of the product is a serious drawback. So, in search of a means for producing a high yield, we decided to follow the reaction in aqueous solution under anaerobic conditions (**Table 1, entries 5-9**).

**Table 1. Synthesis of NRH from NRCl under different conditions.**

| **Entry Reaction conditions** | | **Base** | **Solid support** | **NRCl:Na₂S₂O₄ (mol:mol)** | **Time** | **Yield (%)^{e}** |
|---|---|---|---|---|---|---|
| **1** | Solvent-free^{a} | NaHCO₃ (0.25 g) | SiO₂ (0.25 g) | 1:3.4 | 15 min | 0 |
| **2** | Solvent-free^{a} | NaHCO₃ (0.25 g) | Al₂O₃ (0.25 g) | 1:3.4 | 15 min | 0 |
| **3** | Solvent-free/Solution^{b} | NaHCO₃ (0.25 g) | SiO₂ (0.25 g) | 1:3.4 | 15+30 min | 15 |
| **4** | Solvent-free/Solution^{b} | NaHCO₃ (0.25 g) | Al₂O₃ (0.25 g) | 1:3.4 | 15+30 min | 17 |
| **5** | Solution^{c} | NaHCO₃ (1.2 M) | - | 1:2.0 | 3 h | 42 |
| **6** | Solution^{c} | NaHCO₃ (1.2 M) | - | 1:2.5 | 3 h | 50 |
| **7** | Solution^{c} | NaHCO₃ (1.2 M) | - | 1:2.7 | 3 h | 55 |
| **8** | Solution^{c} | NaHCO₃ (1.2 M) | - | 1:3.0 | 3 h | 54 |
| **9** | Solution" | NaHCO₃ (1.2 M) and Na₂CO₃ (0.2 g) | - | 1:2.7 | 15 h | 51^{f} |

| | | | | | | |
|---|---|---|---|---|---|---|
| ^{a} NRCl (0.1 g), Na₂S₂O₄ (0.2 g), base and solid support were placed in a mortar and ground. ^{b} NRCl (0.1 g), Na₂S₂O₄ (0.2 g), base and solid support were placed in a mortar and ground for 15 min, then all the reaction mixture were added to 8 mL of NaHCO₃ (1 M) solution and stirred for 30 min. ^{c}The reaction of NRCl (0.5 g) and Na₂S₂O₄ (0.8 g) was carried out in 20 mL of NaHCO₃ (1.2 M) solution under N₂ atmosphere at pH 8.1. ^{d}The reaction of NRCl (0.5 g) and Na₂S₂O₄ (0.8 g) was carried out in a solution of 20 mL of NaHCO₃ (1.2 M) and 0.2 g of Na₂CO₃ under N₂ atmosphere at pH 8.5. ^{e} The yields refer to the isolated pure products. ^{f}The product contains 12% of dihydronicotinamide impurity based on ¹H NMR. | | | | | | |

### Results and discussion

In the present study, we introduce a direct procedure for the scalable synthesis of NRH by using commercially available β-NRCl. The reaction was carried out in an aqueous solution of NaHCO₃ and Na₂S₂O₄ under a nitrogen atmosphere (**Scheme 4**). We developed a fast, column chromatography method for the purification of the NRH from the reaction mixture that results in 96% purity. Since one of the main applications of NRH is in supplemental beverages, we also investigated the effect of temperature, light, pH, and oxygen of the stability of NRH in aqueous solutions and compared these results to the stability of the NR in similar conditions.

One of the most significant parameters, and one which directly affects the NRH synthesis reaction and the NRH product, is the pH. Adjusting and maintaining a constant pH is important during the course of the reaction. This issue can be understood better by reviewing the mechanism of the reaction (**Scheme 5**). An aqueous solution of Na₂S₂O₄ is unstable under aerobic conditions at pH=7. Therefore, this reaction must be carried out under anaerobic and alkaline conditions. At first, the reaction between NR and S₂O₄²⁻ leads to the formation of a sulfinate intermediate, which is stable at basic conditions. By protonating this compound, its sulfinic acid derivative is formed. This sulfinic acid intermediate is unstable at ambient conditions and converts to NRH via SO₂ leaving. Therefore, it is important to find and establish a pH that will not only stabilize Na₂S₂O₄ but will also protonate the sulfonate intermediate to produce the NRH. Moreover, because NRH has an N-glycoside bond in its structure, it is susceptible to hydrolysis. Consequently, it is necessary to adjust the pH and maintain it precisely during the whole course of the reaction so that NRH is not hydrolyzed.

The minimum pH to stabilize the aqueous solution of Na₂S₂O₄, without decreasing its activity as a reducing agent, is between 8.0-8.5. Initially, we set up the reaction at pH 8.1 by employing a solution of NaHCO₃ (1.2 M). Next, NRCl was dissolved in this solution and Na₂S₂O₄ was gradually added to the reaction mixture, under a nitrogen atmosphere at 0°C. By adding Na₂S₂O₄ to the solution, and throughout its oxidation, the pH decreased. However, the sodium bicarbonate solution with the concentration of 1.2 M was enough to keep the pH constant during the course of the reaction. After adding Na₂S₂O₄, the reaction was carried out at room temperature for 3 hours. Then, the mixture was freeze-dried to obtain a yellow solid. Finally, the residue was purified by short column chromatography with a mixture of basic alumina and silica (methanol as eluent) to obtain the pure NRH product in 55% yield (Table 1, entry 7). The optimized molar ratio between NRCl and Na₂S₂O₄ was 1 to 2.7 and an increase in the amount of sodium dithionite did not improve the product yield (**Table 1, entry 8**). After purification of the crude product, we conducted thin layer chromatography (TLC) with the purified NRH using methanol as a solvent and compared with the pristine NRCl and nicotinamide (NA) (FIG. 1a). All compounds were active at a wavelength of 254 nm. However, by switching the wavelength to 365 nm, we found that only NRH fluoresced. It has been reported that NRH is strongly fluorescent around 340 nm. Because the R_{f} values of NRH and NA were almost the same, the fluorescence property of NRH helped to identify the exact position of this compound during TLC. As shown in FIG. 1a, the purified NRH shows high purity on the TLC plate.

The FT-IR spectrum of the synthesized and purified NRH was studied and compared to the spectrum of the pristine NRCl (**FIG. 2b**). In the FT-IR spectrum of NRH, the existence of a specific peak at 1643 cm⁻¹ can be attributed to the stretching vibration of C=C band, and confirms the reduction of the NR pyridinium ring. Two peaks at 3340 and 3209 cm⁻¹ refer to asymmetric and symmetric stretching bands of NH₂ group in the structure of NRH which implies that the amide group is intact during the course of the reaction. A sharp peak at 1685 cm⁻¹ indicates the stretching C=O band of the amide group. Two peaks at 2920 and 2840 cm⁻¹ confirm the stretching vibration of aliphatic C-H in both the ribose and the dihydronicotinamide rings of the NRH structure. The presence of hydroxyl groups in the structure of NRH is confirmed with a broad peak that appears in the range of 3500 to 3000 cm⁻¹.

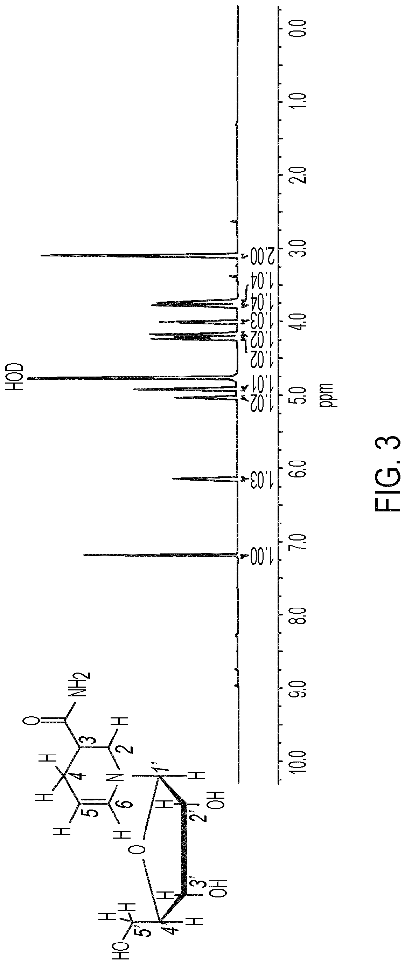

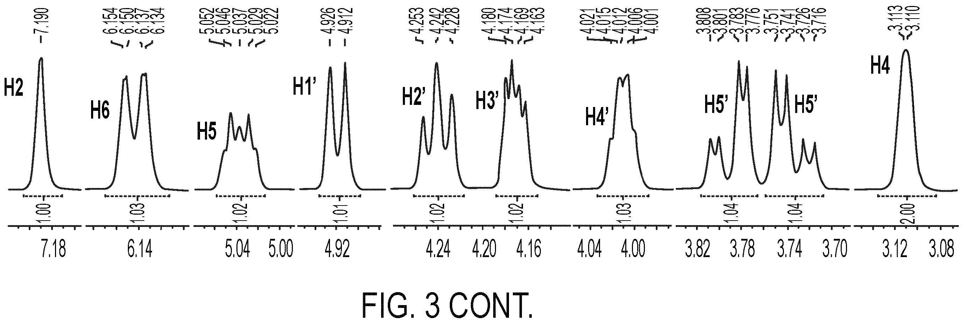

In order to further ensure the successful synthesis of NRH and its purity, we also took ¹HNMR and ¹³CNMR spectra of the purified NRH in deuterated water. The existence of eleven protons that are not exchangeable with D₂O completely confirms the structure of synthesized NRH (**FIG. 3**). The chemical shift of each proton and the corresponding coupling constants are in agreement with the ones previously reported in the scientific literature. The ¹³CNMR and more details are given in **FIG. 12****.**

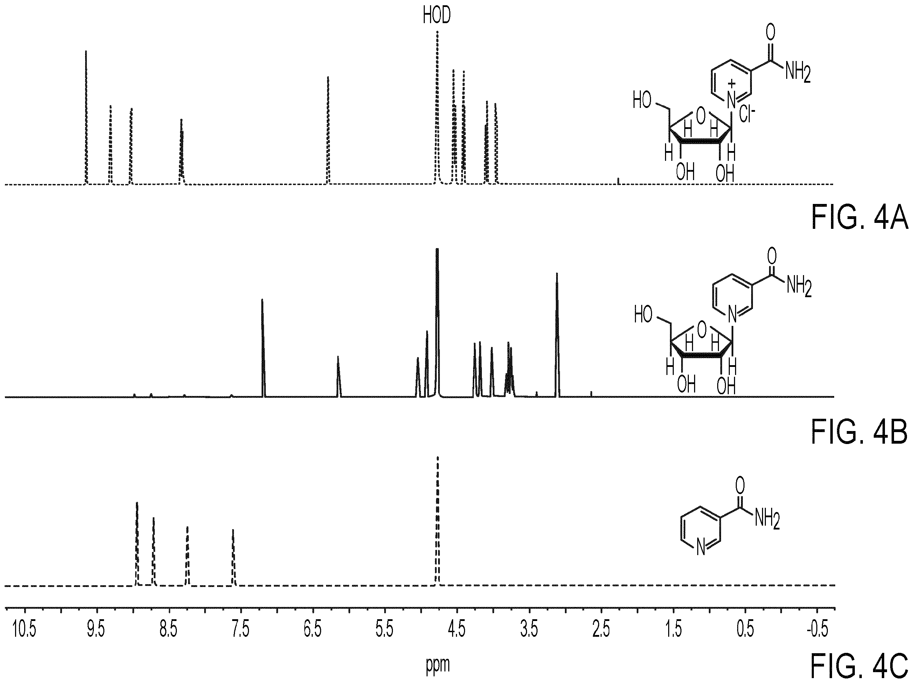

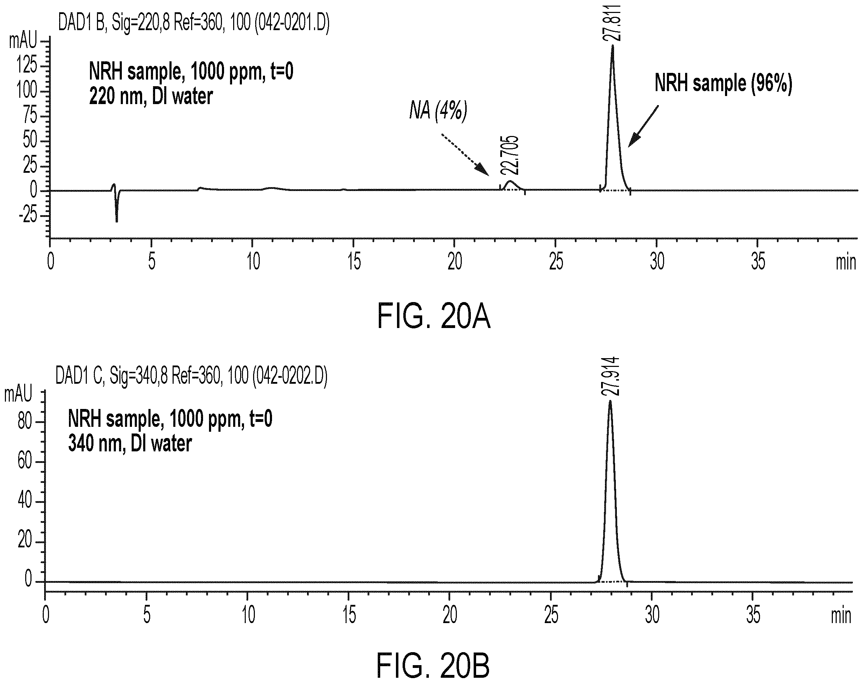

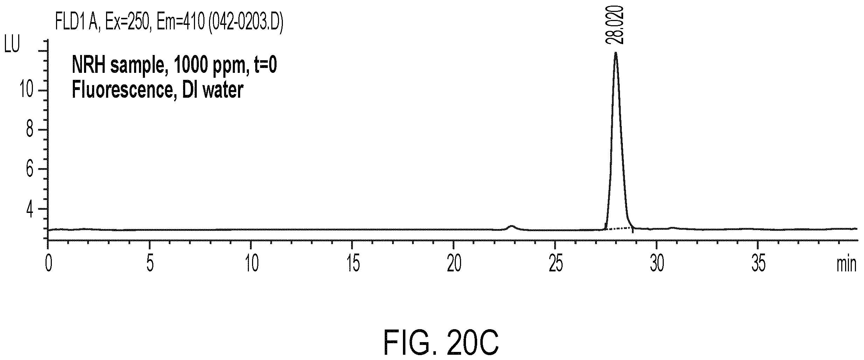

To study the efficiency of the column used for the purification of NRH, we compared the ¹HNMR of purified NRH with the ¹HNMR of pure NRCl and NA (**FIG. 4**). The major impurity in the reaction mixture was unreacted NR and, after purification, we observed no specific NR peak in the ¹HNMR spectrum of purified NRH. By comparing the NRH spectrum with the NA spectrum, we found that there were only trace peaks, thus indicating NA in the ¹HNMR of purified NRH. This signifies that only a trace amount of NR hydrolyzes during the course of the reaction to form NA. We also used reverse phase chromatography HPLC to further confirm the purity of the NRH sample (**FIG. 20**). The HPLC findings agreed with ¹HNMR data and showed both that the purity of NRH was 96% and the impurity of NA was 4%. Interestingly, no NR was detected with HPLC, although NR was present with ¹HNMR. This means that column chromatography on a mixture of basic alumina and silica is very efficient and able to completely purify the NRH product from the remaining NRCl.

Next, we tried to purify crude NRH, using column chromatography on silica and using methanol as the eluent. The results showed that column chromatography on silica alone was not effective in separating NR from NRH. Basic alumina, as a polar surface with many hydroxyl groups on it, can, to a moderate degree, physically separate NR from NRH (by difference of their polarities). Moreover, because basic alumina has negative charges on its surface and NR has a positive charge, it can act as a cationic exchange resin to immobilize NR on its surface. We believe this is the main reason why alumina is a better stationary phase compared to silica for the purification of the NRH. However, we discovered that during the purification of NRH in the column chromatography, basic alumina would clog and result in a very slow flow rate that led to the degradation of NRH. We took steps to easily solved this problem, by mixing basic alumina with silica with the weight ratio of 2:3 respectively. Thus, for the first time, we could quantitatively purify NRH in a preparative mode (without using HPLC) using a common column chromatography method on a mixture of basic alumina and silica, while using methanol as the eluent.

Next, we set up the reduction reaction of NRCl at pH 8.5 by employing a solution of NaHCO₃ and Na₂CO₃ (Table 1, entry 9). The NRCl was dissolved in this solution, then Na₂S₂O₄ was gradually added to the reaction mixture under a nitrogen atmosphere at 0°C. After the addition of Na₂S₂O₄, the reaction was carried out at room temperature for 15 hours. Upon completion of the reaction (followed by TLC), the mixture was freeze-dried to obtain a yellow solid. Finally, the residue was purified by short column chromatography on basic alumina using methanol as the eluent. As a qualitative test, we conducted TLC from this purified NRH and compared with the NR and NA using methanol as a solvent (**FIG. 5a**). The results showed that the NRH obtained was not pure after column chromatography. At this point, we observed that the new impurity was positioned only slightly above the NRH on the TLC. Our observation helped to explain why this contamination cannot be separated from the NRH by common column chromatography. Interestingly, the fluorescence property of this new impurity is similar to NRH, causing both of them to fluoresce at 365 nm on TLC. By studying the literature, we found that it was likely 1,4-dihydronicotinamide (DHNA) formed by hydrolysis of the NRH during the course of the reaction.

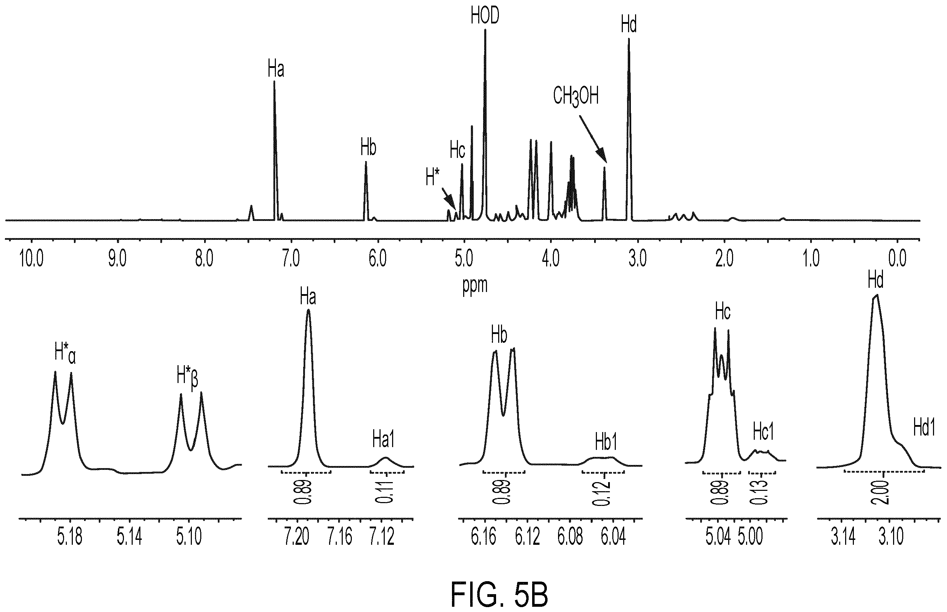

For further evidence, we took ¹HNMR of this sample in D₂O (**FIG. 5b**). The obtained results indicated that there was no residual NRCl in this sample. However, the presence of four peaks with low intensity between 7.5-9 ppm confirmed the existence of a trace amount of NA in the purified NRH. As shown in **FIG. 5b** , four main peaks at 7.19, 6.14, 5.03 and 3.11 ppm strongly confirm a 1,4-dihydronicotinamide ring in the structure of NRH. At the right side of each of the main peaks, there is a peak with lower intensity and a shape is similar to the corresponding main peak. The chemical shifts of these peaks are 7.12, 6.05 and 4.98 ppm, respectively. The integral of each peak is around 0.12 and the integral of each main peak is 0.89.

These results demonstrate that around 12% of DHNA exist in the purified NRH. In other words, this means that by increasing the pH, the reaction rate decreases and the NRH is hydrolyzed under the reaction conditions within 15 hours. It is clear that by hydrolyzing NRH, D-ribose and DHNA are formed simultaneously. As shown in **FIG. 5b** ,the corresponding impurity peaks at 5.19 and 5.10 ppm are attributed to α and β anomeric protons of D-ribose, respectively. The other impurity peaks of ribose appear between 3.65 and 4.65 ppm.

By increasing the pH from 8.1 to 8.5, the reaction rate obviously decreases and, subsequently, there is an increased possibility of side reactions, such as hydrolysis of both NR and NRH.

These observations are in accordance with the reaction mechanism of the reduction of NR to NRH, as seen in **Scheme 5.** In this mechanism, the reaction involves a sulfinate intermediate which is stable in alkaline conditions and, consequently, it is not easily protonated to produce the NRH. In the process of NRH synthesis, thiosulfate and bisulfite anions are formed as the products of hydrolysis and oxidation of dithionite. These anions, with high nucleophilicity, may attack the carbon **1'** in the structure of NRH and substitute instead of DHNA. This would indirectly increase the rate of NRH hydrolysis. By increasing the pH, the possibility of NRH hydrolysis decreases, while simultaneously the existence of some anions, which act as the nucleophile, can increase the NRH hydrolysis. The hydrolysis of NRH by these anions is more severe and increases the reaction time of NRH synthesis. Therefore, as the most important parameter, the pH of the reaction must be adjusted to minimize the formation of NA and DHNA to decreasing the reaction time. This is a very significant factor because the R_{f} values of these by-products are close to the R_{f} values of NRH; therefore, they cannot be separated from the NRH product by common column chromatography (FIG. 4a). In the present work, we found that the optimized pH for the synthesis of NRH from NR was 8.1; at this pH (8.1) we did not observe any DHNA and the amount of NA was negligible.

Thermal stability is very important for drugs and supplements that have the potential for use in food due to the high temperatures that are often needed for industrial processing. In order to study the thermal stability of the NRH, we performed TGA for the pure NRH from 25 to 900 °C and the results were compared to the pristine NRCl. **FIG. 6** shows the TGA thermograms in the range of 25 to 900 °C for NRH and NRCl. As can be seen in **FIGs. 6a & 6b****,** the NRH is clearly more thermally stable than the NRCl. While both NRH and NR show their greatest weight loss at around 218 °C and 910 °C, respectively, NRH shows only around 50% weight loss at this temperature, while NRCl shows around 65% weight loss at this temperature. NRH shows another distinctive weight loss with a peak at around 800 °C which contributes to 30% weight loss. These results confirm that the NRH is more thermally stable compared to the NRCl under a nitrogen atmosphere. It may be due to the existence of chloride ions in the structure of NRCl, that can leave in the form of HCl when the temperature increases.

### Stability study of the NRH and NR aqueous solutions

A stock solution of the freshly synthesized and purified NRH (10,000 ppm) was prepared in deoxygenated DI water. This stock solution was used for preparation of 1000 ppm NRH solutions for stability measurements. The effects of light, pH (buffer), temperature, and oxygen (as air) were investigated during a 60-day period of storage. The remaining NRH concentration was measured using HPLC. This procedure was also used for an NR stability measurement and the results were compared to the NRH samples at the same conditions.

### Study of the stability of NRH in different conditions

NRH is an unstable, sensitive molecule due to its N-glycosidic bond and can undergo degradation, hydrolysis, and oxidation during exposure to high temperatures, nucleophiles, and oxygen. Some of the major products of these degradation reactions for NRH are shown in **Scheme 6.** Studying the NRH degradation under different conditional variables is important for developing new supplements or potential beverage products. Here, we study the effect of light, temperature, oxygen, and pH on the degradation rate of the freshly synthesized and purified NRH in an aqueous solution during a period of 60 days.

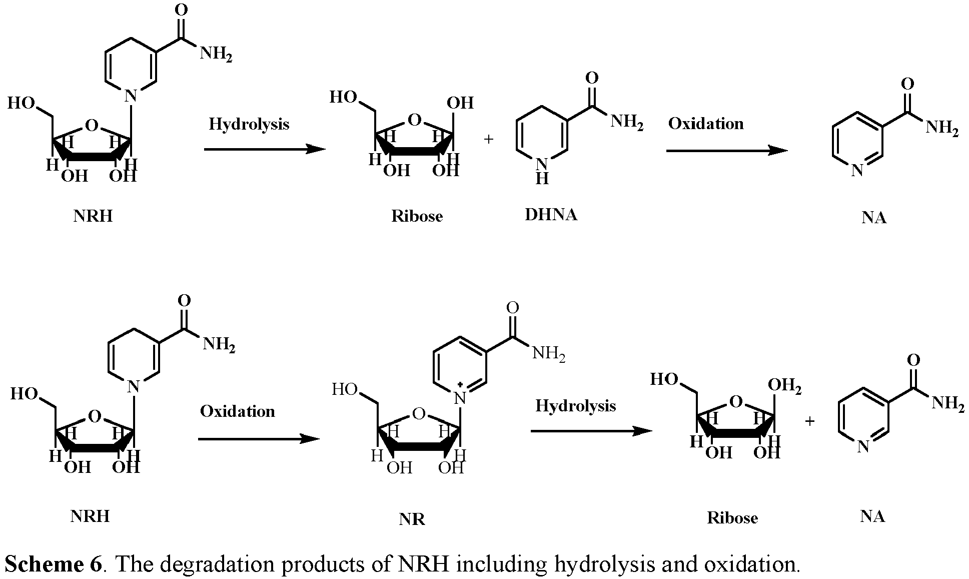

### Effect of oxygen and light

The oxidative sensitivity of the NRH, as a dihydronicotinamide derivative, is well known. As the reduced form of the NR, NRH has a high oxidation potential. In order to study the effect of air, as well as ambient light, on the stability of NRH in an aqueous solution, we prepared solutions of freshly purified NRH (by column chromatography) in DI water in air and under a N₂ blanket as well as in darkness and ambient light. We monitored the NRH concentration over the course of 60 days by HPLC. **FIG. 7a** shows the NRH recovery (%) during the 60 days of storage based on the HPLC results both for samples in the dark/light and in air/under N₂.

From the result shown in **FIG. 7a****,** it is obvious that, while the effect of light is negligible on the stability of NRH compared to the samples that have been kept at dark, oxygen (as air) has a dramatic effect on the stability of the NRH. Samples that have been kept at 25 °C in DI water in darkness show faster degradation reaching 50% in 60 days under air. By contrast, there is around 27% degradation observed for the sample at the same conditions that have been kept under a N₂ blanket. These findings demonstrate that NRH is susceptible to oxidation in the presence of air.

### Effect of temperature

Temperature can have a profound effect on the stability of molecules with an N-glycosidic bond. The NR molecule degradation is temperature dependent following a first order kinetic rule. This is most likely due to the susceptibility of the N-glycosidic bond dissociation because nicotinamide (NA) is a good leaving group. However, the leaving group for NRH is dihydronicotinamide (DHNA) and NRH is not ionic (nitrogen in the ring does not have a positive charge). Therefore, we might anticipate that the NRH should be more stable compared to the NR molecule in terms of spontaneous dissociation of the N-glycosidic bond due to the temperature. However, the oxidative degradation of the NRH also plays an important role for its stability as we showed in previous section.

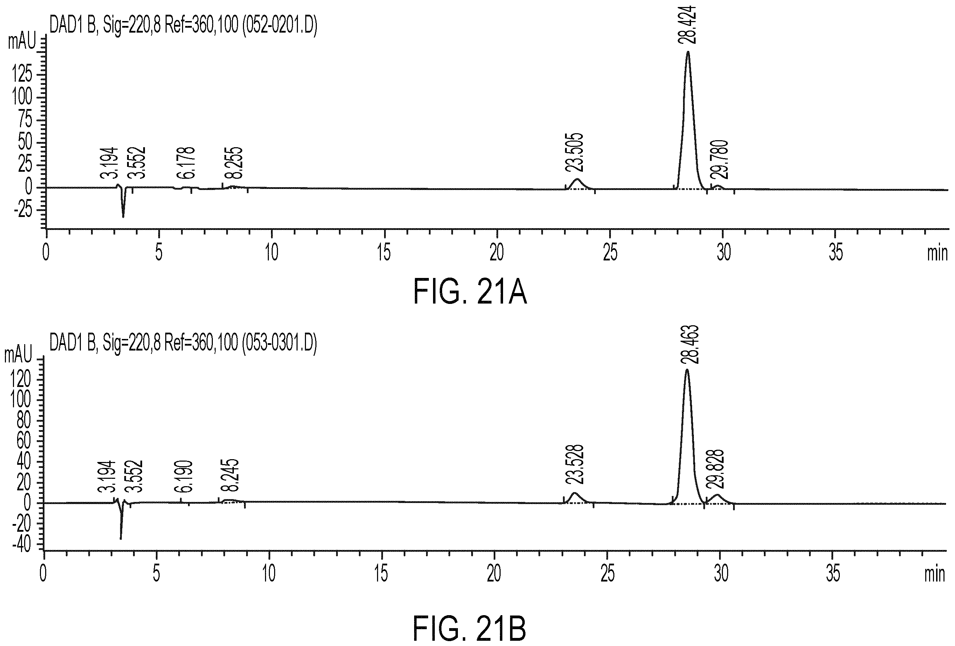

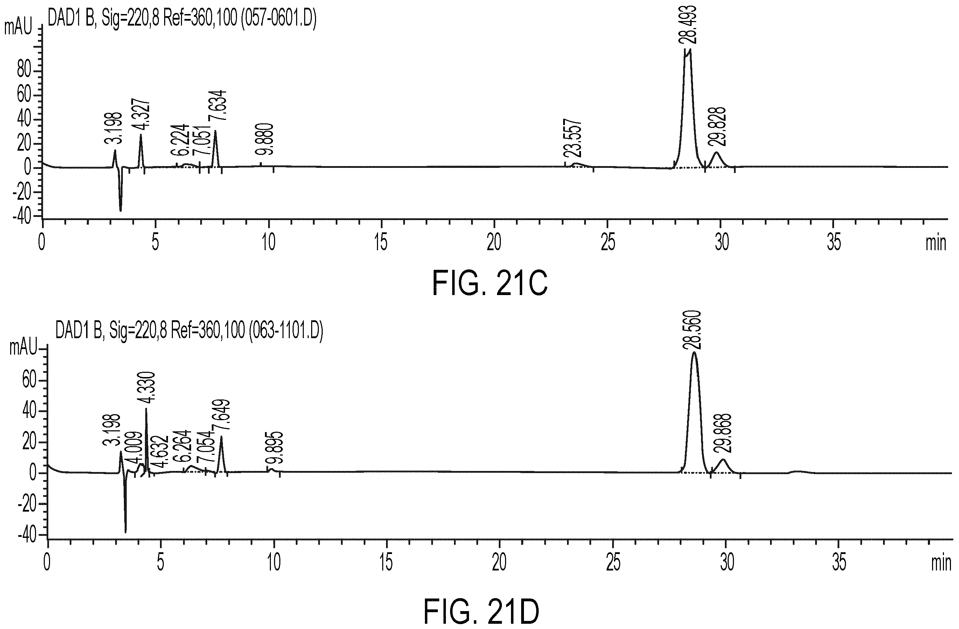

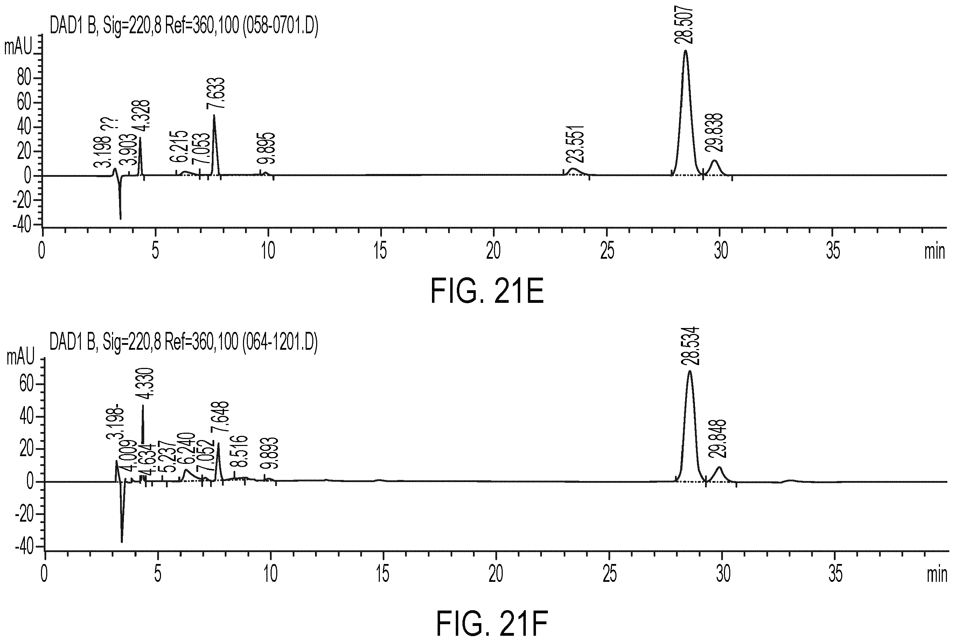

We study the stability of the NRH at two different temperatures both in air and under a N₂ blanket. **FIG. 7b** shows the results of the recovery of the NRH in aqueous solutions kept at 4 °C and 25 °C during 60 days of storage. **FIG. 21** shows the corresponding HPLC chromatograms of some of the samples in 60 days of storage. These results confirm the faster degradation of the NRH at 25 °C compared to 4 °C, and also show the accelerated effect of air on the oxidative degradation of NRH at 25 °C. Samples that have been kept at 4 °C under a N₂ blanket do not show any detectable degradation after 60 days of storage, while samples at 4 °C in air display around 10% degradation in 60 days. For the samples at 25 °C, the degradation is around 27% under a N₂ atmosphere and around 50% in air after 60 days of storage. Pure NRH (as a powder) was stable in a refrigerator, in a sealed tube, for a few months without any degradation.

### Effect of pH (buffer)

Dissociative degradation of the NR molecule is pH independent. The NRH molecule showed relatively good stability in a basic medium (as we also emphasize in the results and discussion) and rapid degradation under acidic conditions during a 10 h monitoring period. We investigated the effect of pH 5, 7, and 9, made using citrate buffer, ammonium acetate buffer, and carbonate buffer, respectively, on the stability of the NRH in the aqueous solutions kept at 25 °C both in air and under N₂, as well as in darkness, for a period of 60 days (**FIG. 7c** and **FIG. 22**). Our results from **FIG. 7c** confirm the complete degradation of NRH in less than one day at pH 5, while samples prepared in the ammonium acetate buffer at pH 7 showed a linear decrease of NRH concentration from around 70% in day one to around 2% in day 30. However, samples prepared in the carbonate buffer at pH 9 showed fair stability both in air (around 45% degradation measured after 60 days) and under N₂ (around 42% degradation measured after 60 days), which agrees with the short term data from others.

Comparing **FIG. 7c** (25 °C, pH 7 air and N₂) with **FIG. 7b** (25 °C, DI water), it is clear that the rapid degradation of the NRH at pH 7 is due to the ammonium acetate salt used to make the buffer. The presence of some anions, such as chloride and acetate, accelerate the oxidation of NRH to NR because the existence of an anion as a counter ion of NR is necessary in this oxidation process. Moreover, the acetate ion may act as a nucleophile and accelerate the NRH hydrolysis. By consuming the acetate anion, the ratio of ammonium to acetate increases and, by hydrolysis of this extra quantity of ammonium, the solution gradually becomes acidic and the NRH hydrolysis rate increases. Therefore, it can be stated that there is a synergistic effect for the degradation of the NRH while at pH 7.0 using a buffer produced with ammonium acetate.

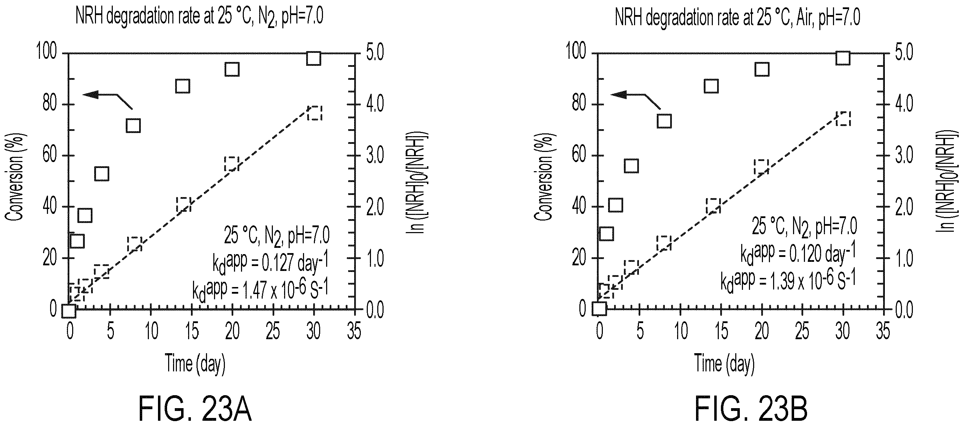

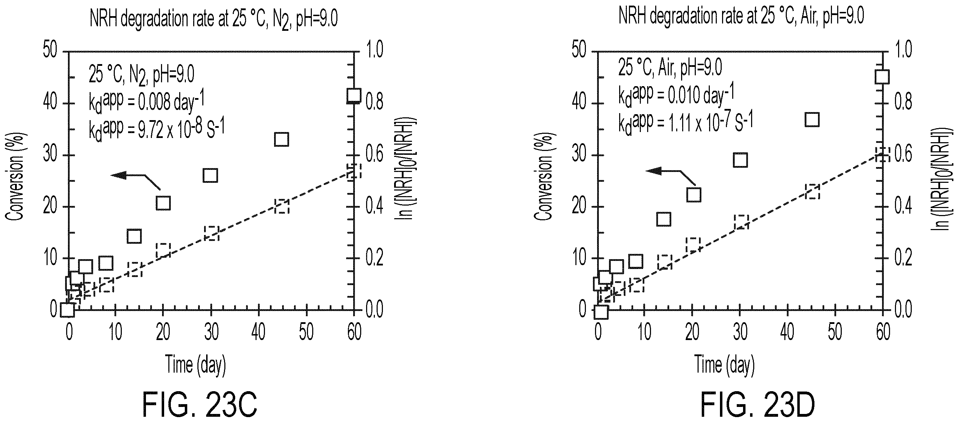

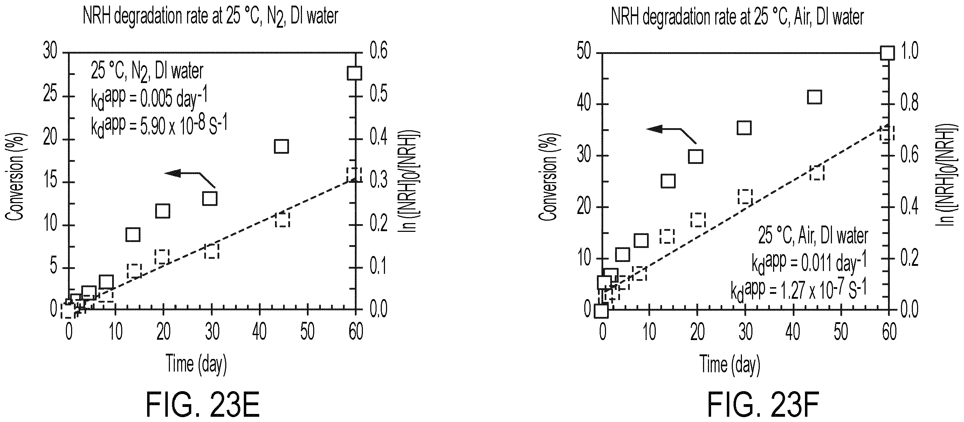

The kinetic graphs for the NRH degradation rate are based on the HPLC data collected during the 60 days of storage for samples with rather fast degradation rates. **FIG. 23** shows the kinetic graphs and the first order degradation rates for NRH samples stored at pH 7.0, at 25 °C, under N₂ and air (**FIGs. 23a and 23b**), samples stored at pH 9.0, at 25 °C, under N₂ and air (**FIGs. 23c and 23d****),** and for NRH samples that have been kept in DI water, at 25 °C, under N₂ and air **(****FIGs. 23e and 23f**). The degradation rate for samples that have been kept at 25 °C in DI water under air is 1.27 × 10⁻⁷ s⁻¹, which corresponds to a half-life of 63 days. By comparison, for the NRH samples that have been kept at 25 °C in DI water under N₂ the degradation rate is 5.90 × 10⁻⁸s⁻¹, which correspond to a half-life of 136 days (**FIG. 23**).

### Comparison of the stability of NRH vs. NR in aqueous solution

NRH is the reduced form of NR and has a much higher biological activity. However, it is essential to compare the stability of NR to NRH. If the NRH is not as stable as NR, the latter would be useful for more applications. Here, we compare the stability of both NR and NRH in aqueous solutions. Since NR stability in aqueous solutions is pH independent, we studied the NR and NRH stability in DI water at 4 °C and 25 °C for a period of 60 days. Figure 6d compares the results of this study for 30 and 60 days.

It is clear from the results of **FIG. 7d** that, at 4 °C, the NR in an aqueous solution is very stable with no detectable degradation after 60 days. However, NRH in an aqueous solution stored in air shows 9% and 10% degradation after 30 and 60 days, respectively, while NRH in an aqueous solution stored under a N₂ blanket is fairly stable with no detectable degradation after 60 days of storage. On the other hand, the NR solution that has been kept at 25 °C (representing the ambient temperature) shows 48% and 68% degradation after 30 and 60 days of storage, while this degradation is 36% and 50% for NRH solutions that have been kept under air after 30 and 60 days and 13% and 27% for and NRH solutions that have been kept under N₂ after 30 and 60 days, respectively. This clearly shows that the thermal degradation is more severe for NR than NRH in aqueous solutions, and storage of NRH away from air improves its stability.

### Conclusions

A convenient, efficient, and scalable procedure for the synthesis and purification of NRH from the commercially available NRCl was developed. In the present method, sodium dithionite was used as a reducing agent under different conditions. While the aerobic solid phase synthesis could not provide high yields, the anaerobic synthesis in a solution of sodium bicarbonate (1.2 M), at pH = 8.1, reached around 55% yield after a 3 h reaction time. For the first time, the purification process was done by using a short, common column chromatography with basic alumina. We found the effect of the reaction's pH was very critical and is optimized at 8.1. Maintaining this pH is essential throughout the entire reaction. We showed that an increase from pH = 8.1 to pH = 8.5 led to an increase in the reaction time and some significant side reactions including hydrolysis of the NRH to ribose and dihydronicotinamide. NMR, FTIR, LC-MS, UV-vis, and HPLC confirmed the structure as well as the high purity of the NRH synthesized by this method. To investigate the effect of temperature, pH, light, and oxygen (air) on the NRH degradation in aqueous solutions, we performed a long-term stability study. Results showed that NRH was oxidized in the presence of oxygen, and it hydrolyzed quickly in acidic conditions. The degradation rate was lower under a N₂ atmosphere, at lower temperatures, and in a basic pH.

## Claims

1. A method of producing 1,4-dihydronicotinamide riboside (NRH), comprising:
providing nicotinamide riboside chloride (NRCl) in a liquid solution;
adding a metal dithionite into the liquid solution under a first temperature; and
reacting the metal dithionite with the nicotinamide riboside chloride (NRCl) in the liquid solution under a second temperature to form a mixture, wherein at least a portion of the mixture comprises the 1,4-dihydronicotinamide riboside (NRH).

2. The method of Claim 1, wherein the nicotinamide riboside chloride (NRCl) comprises β- nicotinamide riboside chloride (β-NRCl).

3. The method of Claim 1, wherein the liquid solution is a basic solution.

4. The method of Claim 3, wherein the basic solution comprises NaHCO₃.

5. The method of Claim 4, comprising maintaining a pH value of the basic solution between 8.0-8.5.

6. The method of Claim 5, wherein the basic solution has a concentration of NaHCO₃ from 1.0 M to 1.5 M.

7. The method of Claim 6, wherein the basic solution has a concentration of NaHCO₃ of about 1.2 M.

8. The method of Claim 1, wherein the metal dithionite comprises sodium dithionite.

9. The method of Claim 1, wherein the first temperature is about 0 °C.

10. The method of Claim 1, wherein the second temperature is room temperature.

11. The method of Claim 1, comprising purging of oxygen from the liquid solution and keeping the liquid solution under an inert gas.

12. The method of Claim 11, wherein the inert gas comprises nitrogen gas.

13. The method of Claim 1, wherein the method further comprises freeze-drying the mixture to obtain a yellow solid powder.

14. The method of Claim 13, wherein the method further comprises purifying the yellow solid powder by column chromatography using a mixture of basic alumina and silica.

15. The method of Claim 14, wherein the basic alumina and the silica have a weight ratio of about 2:3.

16. The method of Claim 15, wherein the 1,4-dihydronicotinamide riboside (NRH) has a purity of at least 90%.

17. The method of Claim 16, wherein the purity of the 1,4-dihydronicotinamide riboside (NRH) is at least 93%.

18. The method of Claim 17, wherein the purity of the 1,4-dihydronicotinamide riboside (NRH) is at least 95%.

19. The method of Claim 18, wherein the purity of the 1,4-dihydronicotinamide riboside (NRH) is at least 96%.

20. The method of Claim 1, wherein a yield of the 1,4-dihydronicotinamide riboside (NRH) is at least 40%.

21. The method of Claim 20, wherein the yield of the 1,4-dihydronicotinamide riboside (NRH) is at least 45%.

22. The method of Claim 21, wherein the yield of the 1,4-dihydronicotinamide riboside (NRH) is at least 50%.

23. The method of Claim 22, wherein the yield of the 1,4-dihydronicotinamide riboside (NRH) is at least 55%.

## Patentansprüche

1. Verfahren zum Herstellen von 1,4-Dihydronicotinamid-Ribosid (NRH), umfassend: Bereitstellen von
Nicotinamid-Ribosidchlorid (NRCI) in einer flüssigen Lösung;
Hinzufügen eines Metalldithionits zu der flüssigen Lösung bei einer ersten Temperatur; und
Reagieren des Metalldithionits mit dem Nicotinamid-Ribosidchlorid (NRCl) in der flüssigen Lösung bei einer zweiten Temperatur, um eine Mischung auszubilden, wobei mindestens ein Anteil der Mischung das 1,4-Dihydronicotinamid-Ribosid (NRH) umfasst.

2. Verfahren nach Anspruch 1, wobei das Nicotinamid-Ribosidchlorid (NRCI) β-Nicotinamid-Ribosidchlorid (β-NRCI) umfasst.

3. Verfahren nach Anspruch 1, wobei die flüssige Lösung eine basische Lösung ist.

4. Verfahren nach Anspruch 3, wobei die basische Lösung NaHCO₃ umfasst.

5. Verfahren nach Anspruch 4, umfassend ein Aufrechterhalten eines pH-Werts der basischen Lösung zwischen 8,0 - 8,5.

6. Verfahren nach Anspruch 5, wobei die basische Lösung eine NaHCOs-Konzentration von 1,0 M bis 1,5 M aufweist.

7. Verfahren nach Anspruch 6, wobei die basische Lösung eine NaHCOs-Konzentration von etwa 1,2 M aufweist.

8. Verfahren nach Anspruch 1, wobei das Metalldithionit Natriumdithionit umfasst.

9. Verfahren nach Anspruch 1, wobei die erste Temperatur etwa 0 °C ist.

10. Verfahren nach Anspruch 1, wobei die zweite Temperatur Raumtemperatur ist.

11. Verfahren nach Anspruch 1, umfassend ein Entfernen von Sauerstoff aus der flüssigen Lösung und ein Halten der flüssigen Lösung unter einem Inertgas.

12. Verfahren nach Anspruch 11, wobei das Inertgas Stickstoffgas umfasst.

13. Verfahren nach Anspruch 1, wobei das Verfahren ferner ein Gefriertrocknen der Mischung umfasst, um ein gelbes festes Pulver zu erhalten.

14. Verfahren nach Anspruch 13, wobei das Verfahren ferner ein Reinigen des gelben festen Pulvers durch Säulenchromatographie unter Verwendung einer Mischung aus basischem Aluminiumoxid und Siliciumdioxid umfasst.

15. Verfahren nach Anspruch 14, wobei das basische Aluminiumoxid und das Siliciumdioxid ein Gewichtsverhältnis von etwa 2 : 3 aufweisen.

16. Verfahren nach Anspruch 15, wobei das 1,4-Dihydronicotinamid-Ribosid (NRH) eine Reinheit von mindestens 90 % aufweist.

17. Verfahren nach Anspruch 16, wobei die Reinheit des 1,4-Dihydronicotinamid-Ribosids (NRH) mindestens 93 % beträgt.

18. Verfahren nach Anspruch 17, wobei die Reinheit des 1,4-Dihydronicotinamid-Ribosids (NRH) mindestens 95 % beträgt.

19. Verfahren nach Anspruch 18, wobei die Reinheit des 1,4-Dihydronicotinamid-Ribosids (NRH) mindestens 96 % beträgt.

20. Verfahren nach Anspruch 1, wobei eine Ausbeute des 1,4-Dihydronicotinamid-Ribosids (NRH) mindestens 40 % beträgt.

21. Verfahren nach Anspruch 20, wobei die Ausbeute des 1,4-Dihydronicotinamid-Ribosids (NRH) mindestens 45 % beträgt.

22. Verfahren nach Anspruch 21, wobei die Ausbeute des 1,4-Dihydronicotinamid-Ribosids (NRH) mindestens 50 % beträgt.

23. Verfahren nach Anspruch 22, wobei die Ausbeute des 1,4-Dihydronicotinamid-Ribosids (NRH) mindestens 55 % beträgt.

## Revendications

1. Procédé de production de 1,4-dihydronicotinamide riboside (NRH), comprenant : la fourniture de
chlorure de nicotinamide riboside (NRCI) dans une solution liquide ;
l'ajout d'un dithionite métallique dans la solution liquide à une première température ; et
la réaction du dithionite métallique avec le chlorure de nicotinamide riboside (NRCI) dans la solution liquide à une seconde température pour former un mélange, dans lequel au moins une partie du mélange comprend le 1,4-dihydronicotinamide riboside (NRH).

2. Procédé selon la revendication 1, dans lequel le chlorure de nicotinamide riboside (NRCI) comprend du chlorure de β-nicotinamide riboside (β-NRCl).

3. Procédé selon la revendication 1, dans lequel la solution liquide est une solution basique.

4. Procédé selon la revendication 3, dans lequel la solution basique comprend NaHCO₃.

5. Procédé selon la revendication 4, comprenant le maintien d'une valeur de pH de la solution basique entre 8,0 et 8,5.

6. Procédé selon la revendication 5, dans lequel la solution basique a une concentration en NaHCO₃ allant de 1,0 M et 1,5 M.

7. Procédé selon la revendication 6, dans lequel la solution basique a une concentration en NaHCO₃ d'environ 1,2 M.

8. Procédé selon la revendication 1, dans lequel le dithionite métallique comprend du dithionite de sodium.

9. Procédé selon la revendication 1, dans lequel la première température est d'environ 0 °C.

10. Procédé selon la revendication 1, dans lequel la seconde température est la température ambiante.

11. Procédé selon la revendication 1, comprenant la purge de l'oxygène de la solution liquide et le maintien de la solution liquide sous un gaz inerte.

12. Procédé selon la revendication 11, dans lequel le gaz inerte comprend de l'azote gazeux.

13. Procédé selon la revendication 1, dans lequel le procédé comprend en outre la lyophilisation du mélange pour obtenir une poudre solide jaune.

14. Procédé selon la revendication 13, dans lequel le procédé comprend en outre la purification de la poudre solide jaune par chromatographie sur colonne à l'aide d'un mélange d'alumine basique et de silice.

15. Procédé selon la revendication 14, dans lequel l'alumine basique et la silice ont un rapport en poids d'environ 2:3.

16. Procédé selon la revendication 15, dans lequel le 1,4-dihydronicotinamide riboside (NRH) a une pureté d'au moins 90 %.

17. Procédé selon la revendication 16, dans lequel la pureté du 1,4-dihydronicotinamide riboside (NRH) est d'au moins 93 %.

18. Procédé selon la revendication 17, dans lequel la pureté du 1,4-dihydronicotinamide riboside (NRH) est d'au moins 95 %.

19. Procédé selon la revendication 18, dans lequel la pureté du 1,4-dihydronicotinamide riboside (NRH) est d'au moins 96 %.

20. Procédé selon la revendication 1, dans lequel un rendement du 1,4-dihydronicotinamide riboside (NRH) est d'au moins 40 %.

21. Procédé selon la revendication 20, dans lequel le rendement du 1,4-dihydronicotinamide riboside (NRH) est d'au moins 45 %.

22. Procédé selon la revendication 21, dans lequel le rendement du 1,4-dihydronicotinamide riboside (NRH) est d'au moins 50 %.

23. Procédé selon la revendication 22, dans lequel le rendement du 1,4-dihydronicotinamide riboside (NRH) est d'au moins 55 %.
